(19) Europäisches Patentamt
European Patent Office
Office européen des brevets

(11) EP 0 736 515 B1

(12) EUROPÄISCHE PATENTSCHRIFT

(45) Veröffentlichungstag und Bekanntmachung des Hinweises auf die Patenterteilung:
**20.01.1999 Patentblatt 1999/03**

(51) Int. Cl.[6]: **C07C 209/86**, C07C 209/84, C07C 211/50

(21) Anmeldenummer: **96104867.5**

(22) Anmeldetag: **27.03.1996**

(54) **Fraktionierung und Reinigung von aromatischen Polyamingemischen und deren Verwendung**
Fractionation and purification of mixtures of aromatic polyamines and their use
Fractionnement et purification de mélanges de polyamines aromatiques et leur utilisation

(84) Benannte Vertragsstaaten:
**BE DE ES FR GB IT NL PT**

(30) Priorität: **07.04.1995 DE 19513147**

(43) Veröffentlichungstag der Anmeldung:
**09.10.1996 Patentblatt 1996/41**

(73) Patentinhaber: **BAYER AG**
**51368 Leverkusen (DE)**

(72) Erfinder:
- **Knöfel, Hartmut, Dr.**
  **51519 Odenthal (DE)**
- **Brockelt, Michael**
  **51379 Leverkusen (DE)**

(56) Entgegenhaltungen:
**EP-A- 0 288 892**
**DE-A- 1 568 087**
**DE-A- 2 528 694**

- **BECKER H. ET AL.: "Organikum" 1976 , VEB DEUTSCHER VERLAG DER WISSENSCHAFTEN , BERLIN XP002005624 * Seite 74 - Seite 82 ***

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 736 515 B1

## Beschreibung

Die Erfindung betrifft ein Verfahren zur Fraktionierung und Reinigung von aromatischen Polyamingemischen und deren Verwendung.

Die Herstellung von aromatischen Polyaminen und Polyamingemischen, insbesondere der Diphenylmethanreihe, wird in zahlreichen Patentmeldungen und Patenten beschrieben, ebenso die Verwendung dieser Produkte. Herausragende Bedeutung kommt dabei der Verwendung dieser Produkte als Rohstoffe für die Herstellung von Isocyanaten zu, in der Regel durch Umsetzung der Polyamingemische mit Phosgen nach den allgemein üblichen und bekannten Methoden.

Die dabei resultierenden Isocyanate bzw. Isocyanatgemische fallen in vielen Fällen aber nicht in der Form und Zusammensetzung an, wie sie auf der Isocyanatstufe bevorzugt weiterverwendet werden, sondern müssen zuvor durch teilweise aufwendige Aufarbeitungs- und Trennverfahren in die verwendungsgerechte Form übergeführt werden. Geeignete Polyaminvorstufen, die weniger aufwendig in die Isocyanatverwendungsformen gebracht werden können, sind in vielen Fällen verfahrenstechnisch schwierig oder gar nicht zugänglich oder wirtschaftlich unattraktiv herzustellen.

Beispielhaft ist die Gewinnung des für die Herstellung hochwertiger Polyurethanwerkstoffe wichtigen 4,4'-Diisocyanato-diphenylmethans, dessen Aminvorstufe in der Regel aus Anilin und Formaldehyd nur gemeinsam mit Isomeren, insbesondere dem 2,4'-Isomeren, und höherfunktionellen Polyaminen gewonnen werden kann. Diese Bestandteile sind zwar die Grundlage für ebenfalls begehrte Isocyanate, doch ist die Auftrennung der Rohisocyanate in die für die Weiterverwendung geeigneten Isocyanate bzw. Isocyanatgemische nicht einfach.

In der Regel werden zunächst ein Teil der Zweikernverbindungen von dem Rest abgetrennt. Anschließend wird aus der Zweikernfraktion in einem viele Trennstufen erfordernden zweiten Destillationsschritt das 4,4'-Diisocyanato-diphenylmethan von den anderen Isomeren befreit.

Das 2,4'-Isomere in angereicherter Form hat selbst in neuerer Zeit zunehmende Bedeutung als Polyurethanrohstoff erlangt, und kann nur mit beträchtlichem destillativen Aufwand gegenüber dem 4,4'-Isomeren angereichert und von dem gegebenenfalls vorhandenen 2,2'-Isomeren befreit werden.

Isomerentrennverfahren oder Anreicherungsverfahren innerhalb der Fraktion der höherkernigen Homologen bzw. der höherfunktionellen Bestandteile der Amine wie auch der Isocyanate der Diphenylmethanreihe sind praktisch nicht bekannt.

Zunehmendes Interesse findet auch das 4,4'-Diamin-diphenylmethan als Rohstoff für das Di-(4-isocyanatocyclohexyl)-methan, die kernhydrierte Form des 4,4'-Diisocyanato-diphenylmethans, wobei die Bereitstellung geeigneter aromatischer Polyamingemische für die Hydrierstufe mit einem möglichst hohen Gehalt an 4,4'-Diamino-diphenylmethan bei gleichzeitig einem möglichst geringen Anteil an 2,4'-Diamino-diphenylmethan sehr aufwendig ist.

Es ist bekannt, daß Amine durch partielle Überführung in ihre Salze in bestimmten Fällen getrennt werden können, wobei u.a. die unterschiedlichen Basenstärken genutzt werden. Dabei handelt es sich in der Regel um Monoamine mit stark unterschiedlichen Basenstärken.

Auch für aromatische Polyamingemische, insbesondere der Diphenylmethanreihe, sind solche Disproportionierungseffekte in zweiphasigen Systemen bereits beschrieben (DE-A 2238319 und DE-A 2528694).

Die Effekte sind infolge der in einem solchen Gemisch vorhanden zahlreichen Komponenten, deren Aminogruppen sich vom Typ her - praktisch alle sind Arylaminogruppen - kaum unterscheiden, nicht besonders groß und ausgeprägt, um für eine direkte Nutzung mit einfachen Mitteln interessant zu sein.

Diese Aufgabe konnte durch das erfindungsgemäße Verfahren, welches aufgrund der erfindungsgemäßen Ausführung überraschend hohe Trennleistung bei der Fraktionierung von aromatischen Polyamingemischen, insbesondere der Diphenylmethanreihe, erzielt, und dabei in der Wirkung weit über die bekannten Effekte des Standes der Technik hinausgeht, gelöst werden.

Bei der erfindungsgemäßen Fraktionierung von aromatischen Polyamingemischen werden andere, unterschiedlich zusammengesetzte Polyamingemische gewonnen.

Bei diesen abgeleiteten Polyamingemischen kann es sich um solche handeln, die auf bekannten Synthesewegen nur sehr aufwendig zugänglich sind. Dabei kann es sich auch um Polyamingemische handeln, die für eine vereinfachte Herstellung der Isocyanate besser geeignet sind, als die bekannten und technisch gut herzustellenden Polyamingemische, indem sie z.B. auf der Isocyanatstufe schwierig durchzuführende Isomerentrennungen durch entsprechende Isomerenanreicherungen auf der Aminstufe vorwegnehmen. Solche Gemische können auch völlig neuartige, weil nach dem Stand der Technik nicht darstellbare Polyamingemische sein, die zu völlig neuartigen Isocyanaten führen.

Andererseits kann das erfindungsgemäße Verfahren dazu genutzt werden, aus beliebigen, d.h. auch aus wiedergewonnenen Polyamingemischen, die sich durch Verunreinigung oder durch nichtstatistische, d.h. selektive Verluste bei einzelnen Komponenten bei der Wiedergewinnung von den ursprünglich eingesetzten Polyaminen oder Isocyanaten unterscheiden, Produktfraktionen zu gewinnen, welche dem Standard oder den Ausgangspolyaminen entsprechen.

Schließlich kann das erfindungsgemäße Verfahren dazu genutzt werden, synthesebedingte und im Endprodukt

unerwünschte Neben- und Zwischenprodukte, mitzufraktionieren und in einer Produktfraktion ab - und in einer anderen entsprechend anzureichern, gegebenenfalls in einer eigenen Fraktion auszuschleusen.

Es handelt sich bei der vorliegenden Erfindung uni ein breit anwendbares Verfahren, mit welchem die Aufgabe der Fraktionierung und Reinigung von aromatischen Di- und Polyamingemischen, insbesondere der Diphenylmethanreihe gelöst werden kann.

Aufgabe war es, ein Verfahren zur Verfügung zu stellen, das es gestattet, aromatische Polyamingemische in einfacher Weise zu fraktionieren bzw. zu reinigen, so daß Isomere in reiner Form oder in angereicherter Form anfallen.

Gegenstand der Erfindung ist ein Verfahren zur Fraktionierung und Reinigung von aromatischen Polyamingemischen, insbesondere von Polyamingemischen der Diphenylmethanreihe, welches dadurch gekennzeichnet ist, daß man

a) das Ausgangspolyamingemisch (A) in einem zweiphasigen System, bestehend aus (i) einer hydrophoben Lösungsmittelphase (B), die im wesentlichen aus hydrophobem Lösungsmittel und gegebenenfalls einem aromatischen Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, und gegebenenfalls Polyaminen besteht, und (ii) einer wäßrigen Phase (C), bestehend im wesentlichen aus Wasser, einer starken Säure und zumindest teilweise in der Salzform vorliegendem Hilfsamin, sowie gegebenenfalls zumindest teilweise in der Salzform vorliegenden Polyaminen, unter Zuhilfenahme einer, nach dem Gegenstromprinzip arbeitenden Extraktionsstufe (3) unter Durchmischung der Phasen verteilt, indem man das Ausgangspolyamingemisch vorzugsweise über die organische Phase (B) in die Extraktionsstufe (3) einbringt, die diese Extraktionsstufe verlassende organische Phase (D), zumindest teilweise, gegebenenfalls nach Durchlaufen einer Waschstufe (7.0), in einer mehrstufigen Destillation (7.1), (7.2) in eine erste, in der Extraktionsstufe (3) wiederverwendeten Fraktion (E), bestehend im wesentlichen aus hydrophobem Lösungsmittel und gegebenenfalls Hilfsamin, eine zweite Fraktion (F), bestehend im wesentlichen aus Hilfsamin und gegebenenfalls hydrophobem Lösungsmittel, und einen Destillationsrückstand (G), bestehend im wesentlichen aus einer ersten Polyaminfraktion, auftrennt und die die Extraktionsstufe (3) verlassende wäßrige Phase (H),

b) gegebenenfalls zumindest teilweise über eine zwischengeschaltete Extraktionsstufe(4)

c) in eine Extraktionsstufe (5) leitet, in welcher nach dem Prinzip der Gegenstromextraktion eine Extraktion der wäßrigen Phase mit einer aus hydrophobem Lösungsmittel und Hilfsamin bestehenden Lösungsmittelphase (J) erfolgt, und die an Polyamin verarmte wäßrige Phase (K) resultiert, und

d) die in der Extraktionsstufe (5) anfallende organische Phase (L) zumindest teilweise, gegebenenfalls nach Durchlaufen einer Waschstufe (8.0), in einer mehrstufigen Destillation (8.1), (8.2) in eine erste Fraktion (M), bestehend im wesentlichen aus hydrophobem Lösungsmittel und gegebenenfalls geringen Anteilen an Hilfsamin, und eine zweite Fraktion (N) bestehend im wesentlichen an Hilfsamin und gegebenenfalls geringen Anteilen an hydrophobem Lösungsmittel und einen Destillationsrückstand (O), bestehend im wesentlichen aus einer zweiten Polyaminfraktion, auftrennt und

e) die in der Extraktionsstufe (5) anfallende wäßrige Phase (K) zumindest teilweise,

f) gegebenenfalls über eine zwischengeschaltete Destillationsstufe (9)

g) in eine Extraktionsstufe (6) leitet und im Gegenstrom mit einer organischen Phase (P) extrahiert, bestehend im wesentlichen aus hydrophobem Lösungsmittel und gegebenenfalls höchstens soviel Hilfsamin, daß in (6) eine gegenüber der eingesetzten wäßrigen Phase (K) an Arylamin verarmte wäßrige Phase (Q) resultiert, die man

h) gegebenenfalls zumindest teilweise über eine zwischengeschaltete Destillationsstufe (9)

i) mit dem gegebenenfalls vorhandenen Rest von (Q) vereinigt und als Mengenstrom (C) wiederverwendet, indem man Mengenstrom (C)

j) gegebenenfalls zumindest teilweise über eine vorgeschaltete Extraktionsstufe (2)

k) gegebenenfalls nach Zugabe von Hilfsamin und/oder Wasser der Extraktionsstufe (3) zuführt und

l) die in der Extraktionsstufe (6) anfallende organische Phase (R) gegebenenfalls nach Zugabe von weiterer, im wesentlichen aus Hilfsamin bestehender organischer Phase aus Mengenstrom (S) als Extraktionsmittelphase (J) der Extraktionsstufe (5) zuführt.

Bevorzugt wird das Verfahren so durchgeführt, daß man die in der Extraktionsstufe (3) anfallende wäßrige Phase (H) zumindest teilweise in einer nachgeschalteten, nach dem Gegenstromprinzip arbeitenden Extraktionsstufe (4) extrahiert unter Verwendung einer organischen Phase (T) als Extraktionsmittel, bestehend neben hydrophobem Lösungsmittel aus Hilfsamin und gegebenenfalls Polyamin, letzteres vorzugsweise mit der Zusammensetzung des zweiten Teilproduktes (O) und vorzugsweise eingebracht als Teilmenge des Mengenstromes (L), die in Verfahrensstufe (4) resultierende organische Phase (U) dem Mengenstrom (B) und damit der Extraktionsstufe (3) und die resultierende wäßrige Phase (V) der Extraktionsstufe (5) zuführt.

Das erfindungsgemäße Verfahren wird besonders bevorzugt so durchgeführt, daß man eine Teilmenge (D") der die Extraktionsstufe (3) verlassenden organischen Phase (D) abtrennt und in einer vorgelagerten, vorzugsweise mehrstufigen, Extraktionsstufe (2) mit zumindest einer Teilmenge, vorzugsweise mit dem gesamten Mengenstrom (C) im Gegenstrom extrahiert, gegebenenfalls unter Zugabe von Hilfsamin, und den Teilmengenstrom (D") so bemißt, daß dabei ein möglichst weitgehender Übergang des in (D") enthaltenen Polyamins in die die Extraktionsstufe (2) verlassende wäßrige Phase stattfindet, besagte wäßrige Phase, gegebenenfalls nach Zugabe von Wasser aus Mengenstrom (Y) und/oder Hilfsamin und/oder weiterer wäßriger Phase aus Mengenstrom (C), der Extraktionsstufe (3) zuführt, die in (2) anfallende organische Phase (Z), bestehend im wesentlichen aus hydrophobem Lösungsmittel und gegebenenfalls Hilfsamin, ebenfalls der Extraktionsstufe (3) zuführt und als Bestandteil der organischen Phase (B) als Lösungsmittel für das Ausgangspolyamin (A) verwendet.

Kennzeichnend für die Ausführung des erfindungsgemäßen Verfahrens ist, daß der wiederverwendete Mengenstrom (C) einen höheren Protonierungsgrad aufweist als die die vorletzte Extraktionsstufe (4) verlassende wäßrige Phase (K), gegebenenfalls bis zu 100 %.

Eine weiter verbesserte und daher bevorzugte Ausführungsform des erfindungsgemäßen Verfahrens, wird dadurch erreicht, daß man die die Extraktionsstufe (5) verlassende wäßrige Phase (K) vor ihrer Wiederverwendung zumindest teilweise und/oder die die Extraktionsstufe (6) verlassende wäßrige Phase (Q) vor ihrer Wiederverwendung zumindest teilweise destillativ (9) von einem Teil (X) des in ihr enthaltenen Wassers befreit, dieses gegebenenfalls zum Waschen (7.0) des der destillativen Aufarbeitung (7.1), (7.2) zugeführten Teiles der die Extraktionsstufe (3) verlassenden organischen Phase (D) und/oder zum Waschen (8.0) des der destillativen Aufarbeitung (8.1), (8.2) zugeführten Teiles der die Extraktionsstufe (5) verlassenden organischen Phase (L) zwecks Entfernens von Säurespuren verwendet, das hierbei anfallende Wasser (Y) an geeigneter Stelle in die wäßrige Phase zurückführt, die resultierende konzentrierte wäßrige Phase mit dem gegebenenfalls verbliebenen Resten von (K) und (Q) vereinigt und als Mengenstrom (C) der Wiederverwendung zuführt.

Als Hilfsamin wird vorzugsweise Anilin eingesetzt und als Polyamingemisch der Diphenylmethanreihe bevorzugt ein Polyamingemisch, wie es bei der säurekatalysierten Anilin/Formaldehyd-Kondensation anfällt.

Die derart behandelten Polyamingemische, also die mit dem erfindungsgemäßen Verfahren erzeugten Fraktionen werden zur Herstellung der entsprechenden aromatischen Polyisocyanatgemische und zur Herstellung von Polyurethankunststoffen verwendet.

Außerdem können die nach dem erfindungsgemäßen Verfahren erzeugten Fraktionen zur Herstellung entsprechender kernhydrierter Polyamine oder als Vernetzer und als Epoxidhärter verwendet werden.

Die aus den fraktionierten Polyamingemischen hergestellten entsprechenden Polyisocyanate werden bevorzugt zur Herstellung von PU-Schaumstoffen eingesetzt.

Ausgangsgemische sind beispielsweise technische Arylamingemische, wie sie bei der Herstellung aus den Ausgangsverbindungen oder wie sie bei der Wiedergewinnung anfallen.

Beispiele von Ausgangsarylamingemischen, für deren Fraktionierung und Reinigung das erfindungsgemäße Verfahren besonders geeignet ist, sind

1. Polyamingemische der Diphenylmethanreihe, wie sie bei der Kondensation und säurekatalysierten Umlagerung von Anilin mit Formaldehyd entstehen,

2. Polyamingemische der Diphenylmethanreihe, wie sie beider säurekatalysierten Kondensation von substituierten Anilinen mit Formaldehyd anfallen,

3. Polyamingemische der Diphenylmethanreihe, wie sie bei der Mischkondensation von substituierten Anilinen untereinander und/oder mit Anilin mit Formaldehyd anfallen,

4. Polyamingemische der Diphenylmethanreihe, wie sie bei der Kondensation, auch der Mischkondensation von

substituierten Anilinen und/oder Anilin mit Aldehyden und/oder Ketonen anfallen,

5. Polyamingemische der Diphenylmethanreihe, wie sie beider Nitrierung und anschließenden Reduktion von Di- und/oder Polyarylmethanen und/oder substituierten Di- und/oder Polyarylmethanen entstehen; unter Polyarylmethanen werden hier insbesondere die Benzylhomologen des Diphenylmethans verstanden,

6. Polyamingemische der Diphenylmethanreihe, wie sie bei der Kondensation von Monoarylmonoaminen (z.B. Anilin, substituierte Aniline) und/oder Monoaryldiaminen (Phenylendiamine, substituierte Phenylendiamine) mit Aldehyden, Ketonen, insbesondere Formaldehyd, und säurekatalysierter Umlagerung entstehen und

7. Polyamingemische der Triphenylmethanreihe, wie sie z.B. bei der Nitrierung und anschließenden Reduktion von Triphenylmethan, insbesondere alkylsubstituierten Triphenylmethanen und seinen höherkernigen, insbesondere Benzylhomologen entstehen.

Bei den zum Einsatz gelangenden hydrophoben Lösungsmitteln handelt es sich um inerte Lösungsmittel des Siedepunktbereiches von 30 - 280°C, vorzugsweise von 80 - 200°C, wie beispielsweise Chlorbenzol, Dichlorbenzol, Benzol, Toluol, Ethylbenzol, Cumol, Xylol, Dichlorethan, Chloroform und Tetrachlorkohlenstoff. Vorzugsweise werden Xylole, d.h. technische Xylolgemische, insbesondere o-Xylol, Toluol, Ethylbenzol, Cumol und Chlorbenzol eingesetzt.

Bevorzugt werden solche Lösungsmittel verwendet, die ein gutes Lösungsvermögen für die eingesetzten Polyamingemische aufweisen.

Bei den eingesetzten Säuren handelt es sich um wasserlösliche Protonsäuren mit einem unter 2,5 , vorzugsweise unter 1,5 liegenden pKA-Wert. Beispiele hierfür sind Salzsäure, Bromwasserstoffsäure, Schwefelsäure, Trifluoressigsäure, Methansulfonsäure oder Phosphorsäure.

Bevorzugt eingesetzt werden Salzsäure und Methansulfonsäure. Die genannten Säuren können auch im Gemisch mit sauren oder neutralen Salzen derartiger Säuren, wie z.B. den entsprechenden Ammoniumsalzen oder auch den entsprechenden Alkalisalzen, eingesetzt werden. Die genannten Säuren werden nicht in freier Form eingesetzt, sondern liegen in dem erfindungsgemäßen Kreislaufsystem in Form der entsprechenden Ammoniumsalze der in den wäßrigen Kreislaufsystem befindlichen Basen vor. Das sind in der Regel Polyamingemische von der Art der Ausgangsgemische und/oder die verwendeten Hilfsamine.

Als Hilfsamin finden in der Regel Monoarylamine, wie z.B. Anilin und/oder am Kern und/oder am Stickstoff alkylsubstituierte Anilinderivate, Verwendung. Bevorzugt werden primäre Aniline eingesetzt, besonders bevorzugt ist Anilin.

Das erfindungsgemäße Verfahren kann sowohl diskontinuierlich als auch kontinuierlich durchgeführt werden. Bevorzugte Ausführungsform ist die kontinuierliche Arbeitsweise. Dabei wird das Verfahren in allen Stufen unter dem Eigendruck des Systems und vorzugsweise in einer Inertgasatmosphäre (Stickstoff) durchgeführt.

Das erfindungsgemäße Verfahren kann zur Steigerung des Anreicherungs-bzw. korrespondierenden Abreicherungseffektes mit jeder der anfallenden Produktfraktionen wiederholt werden.

Das erfindungsgemäße Verfahren kann sowohl mit drei (Abb. 1) als auch mit vier (Abb. 2 und Abb. 3) oder mit fünf Extraktionsstufen (Abb. 4) durchgeführt werden.

Die in Abb. 1-4 dargestellten Fließdiagramme dienen der weiteren Erläuterung des erfindungsgemäßen Verfahrens. In diesen Abbildungen bedeuten:

(1) einen Tank für das Ausgangsarylamingemisch

(2) eine vorgeschaltete Extraktionsstufe

(3) eine erste Extraktionsstufe

(4) eine (zwischengeschaltete) zweite Extraktionsstufe

(5) eine (vorletzte) dritte Extraktionsstufe

(6) eine (letzte) vierte Extraktionsstufe

(7) eine Aufarbeitungsstufe bestehend aus

(7.0) einer Waschstufe

(7.1) einer ersten Destillationsstufe einer Mehrstufendestillation

(7.2) einer letzten Destillationsstufe einer Mehrstufendestillation

(8) eine weitere Aufarbeitungsstufe bestehend aus

(8.0) einer Waschstufe

(8.1) einer ersten Destillationsstufe einer weiteren Mehrstufendestillation

(8.2) einer letzten Destillationsstufe einer weiteren Mehrstufendestillation

(9) einen Wasserverdampfer

(10) einen Tank für ein Verfahrensprodukt

(11) einen Tank für ein weiteres Verfahrensprodukt

Die Bezugszeichen A - Z bezeichnen die Mengenströme auf die nachstehend und in den Beispielen Bezug genommen wird.

Die vorgeschaltete Extraktionsstufe (2) wird im allgemeinen als mehrstufig wirkender Extraktor ausgeführt.

Bei der Extraktionsstufe (3) handelt es sich im einfachsten Fall um eine einstufig wirkende Mischer-Scheidereinheit, vorzugsweise kommen jedoch mehrstufig wirkende Extraktionseinheiten zum Einsatz.

Die gegebenenfalls zwischengeschaltete Extraktionsstufe (4) besteht im einfachsten Falle ebenfalls aus einer Mischer-Scheidereinheit, vorzugsweise kommen jedoch auch hier mehrstufig wirkende Extraktionseinheiten zum Einsatz.

Die Extraktionsstufen (5) und (6) werden im allgemeinen als mehrstufig wirkende Extraktoren ausgeführt.

Die Aufarbeitungsstufen (7) und (8) dienen der Abtrennung der Polyaminfraktionen, welche als Destillationsrückstände anfallen und als Verfahrensprodukte (G) und (O) in den Tanks (10) und (11) isoliert werden, und der Wiedergewinnung des eingesetzten hydrophoben Lösungsmittels und des verwendeten Hilfsamins als Destillate.

Im allgemeinen ist die Wiedergewinnung von hydrophobem Lösungsmittel und Hilfsamin mit einer weitgehenden, gegebenenfalls vollständigen Auftrennung beider Komponenten verbunden und erfolgt unter Gewinnung von zumindest an Hilfsamin weitgehend verarmten, gegebenenfalls von Hilfsamin weitgehend befreiten hydrophobem Lösungsmittel in Form der Destillatströme (E) und (M) einerseits und von zumindest an hydrophobem Lösungsmittel weitgehend verarmten, gegebenenfalls von hydrophobem Lösungsmittel weitgehend befreiten Hilfsamin in Form der Destillatströme (F) und (N), in der Regel zusammengefaßt im Mengenstrom (S).

Es hat sich als zweckmäßig erwiesen, die den Destillationsstufen zugeführten organischen Phasen (D) bzw. (D') und (L) bzw. (L') vor ihrer destillativen Behandlung in vorgelagerten Waschstufen(7.0) und (8.0) durch Extraktion mit Wasser von anhaftenden Säurespuren zu befreien.

Die eigentliche Aufarbeitungsstufe (7) besteht im allgemeinen aus einer wenigstens zweistufigen Mehrstufendestillation, deren erste Stufe (7.1) ein gegenüber dem Zulaufprodukt (D) bzw. (D') von Polyarylamin befreites und an Hilfsamin verarmtes, gegebenenfalls von Hilfsamin befreites hydrophobes Lösungsmittel als Destillat (E) liefert und deren letzte Stufe (7.2) ein gegenüber (D) bzw. (D') von Polyarylamin befreites und an hydrophobem Lösungsmittel verarmtes, gegebenenfalls von hydrophobem Lösungsmittel befreites Hilfsamin als Destillat (F) liefert.

Zusätzlich fällt in der letzten Destillationsstufe (7.2) die im Mengenstrom (D) bzw. (D') enthaltene erste Polyaminfraktion des Ausgangsgemisches (A) als Destillationssumpf (G) an.

Auch die Aufarbeitungsstufe (8) besteht im allgemeinen aus einer wenigstens zweistufigen Mehrstufendestillation, deren erste Stufe (8.1) ein gegenüber dem Zulaufprodukt (L) bzw. (L') von Polyamin befreites und an Hilfsamin verarmtes, gegebenenfalls von Hilfsamin befreites hydrophobes Lösungsmittel als Destillat (M) liefert und deren letzte Stufe (8.2) ein gegenüber (L) bzw. (L') von Polyarylamin befreites und an hydrophobem Lösungsmittel verarmtes, gegebenenfalls von an hydrophobem Lösungsmittel befreites Hilfsamin als Destillat (N) liefert.

Die weitgehend, gegebenenfalls vollständige destillative Auftrennung von hydro phobem Lösungsmittel und Hilfsamin ist bei der Durchführung des erfindungsgemäßen Verfahrens bevorzugt, das gilt insbesondere für die Destillatfraktionen (M) und (E).

Bei der Destillationsstufe (9) handelt es sich um eine Vorrichtung, mit welcher der wäßrigen Phase des Systems oder einem Teilstrom der wäßrigen Phase destillativ Wasser entzogen werden kann.

Ein solcher Schritt ist für die Durchführung des erfindungsgemäßen Verfahrens grundsätzlich nicht erforderlich, wegen der sich ergebenden Vorteile sind die Ausführungsformen unter Einschluß einer Wasserdestillationsstufe (9) jedoch bevorzugt.

Bei der wäßrigen, die Säure enthaltenden Phase liegt praktisch ein geschlossenes Kreislaufsystem vor, so daß die

Stufe (9) grundsätzlich an einer beliebigen Stelle dieses Kreislaufsystems eingeschoben werden kann. Die Position von Stufe (9) in Anschluß an die Extraktionsstufe (6) und vor Eintritt in die Extraktionsstufen (2) bzw. (3) ist die vorteilhafteste und daher die bevorzugte Ausführungsform.

Die entnommene Wassermenge (X) wird gegebenenfalls nach ihrer Aufteilung in Teilströme und deren unterschiedlicher Verwendung, in Form des Mengenstromes (Y) insgesamt oder in Teilströmen dem System an geeigneter Stelle wieder zugeführt, so daß ein erweitertes und gegebenenfalls verzweigtes in sich geschlossenes wäßriges Kreislaufsystem entsteht.

Dieses schließt auch die Waschstufen (7.0) und/oder (8.0) mit ein. Letztere sind ein oder mehrstufig wirkende nach dem Gegenstromprinzip arbeitende Extraktionsstufen. In Waschstufe (7.0) wird die organische Phase (D) bzw. (D') mit einem Teilstrom von (X) von anhaftenden Säurespuren befreit, in der Waschstufe (8.0) geschieht das Analoge mit der organischen Phase (L) bzw. (L') unter Verwendung eines anderen Teilstromes von (X).

Das mit hydrophobem Lösungsmittel und Hilfsamin contaminierte Destillat (X) ist für die Waschstufen (7.0) und (8.0) bestens geeignet. Die resultierenden Waschwässer weisen in der Regel eine sehr viel geringere Säurekonzentration auf als der eigentliche Säurekreislauf, so daß diese problemlos in Form des Mengenstromes (Y) oder seiner Teilströme recyclisiert werden können, gegebenenfalls kann eine Destillatteilmenge von (X) an den Waschstufen vorbei nach (Y) geführt und zur Aussteuerung eines unterschiedlichen Wassergehaltes in den einzelnen Extraktionsstufen verwendet werden.

Die praktisch quantitative Kreislaufführung der verwendeten Säure ermöglicht den Einsatz kostspieliger Säuren wie z.B. Methansulfonsäure, die wiederum wegen ihrer geringeren Korrosionsneigung die Verwendung kostengünstiger Werkstoffe in den Apparaten des erfindungsgemäßen Verfahrens gestattet.

Es hat sich als zweckmäßig erwiesen, den Säuregehalt der wäßrigen Phase, unabhängig von dem sich in der wäßrigen Phase eines zweiphasigen Systems einstellenden unterschiedlichen Amingehalt, über eine sogenannte "Molarität" zu definieren.

Die Molarität wird festgelegt als theoretische Konzentration von zu 100 % protoniertem Amin (d.h. gleiche Anzahl von Säure- und Aminäquivalenten) in einem rechnerisch um den Anteil an nicht protoniertem Amin verminderten Volumen an wäßriger Phase gemäß der Formel

$$\text{"Molarität"} = \frac{\text{Mol 100 \% protoniertes Amin}}{\text{Vol. wäßr. Phase - Vol. unprot. Amin}}$$

Die so definierte Molarität kann Werte bis 6 annehmen und wird je nach der der jeweiligen Ausführungsform zugrunde liegenden - hier produktbezogenen - Trennaufgabe in diesem Bereich gezielt variiert.

Auch innerhalb einer Ausführungsform des erfindungsgemäßen Verfahrens ist es gegebenenfalls vorteilhaft, die einzelnen von der wäßrigen Phase durchlaufenen Verfahrensstufen, insbesondere die Extraktionsstufen (2) bis (6) mit unterschiedlicher Molarität in der wäßrigen Phase zu betreiben, indem der wäßrigen Phase zwischen den einzelnen Stufen Wasser entzogen oder zugeführt wird.

Nach oben wird dieser Arbeitsbereich praktisch begrenzt einerseits durch die zunehmende Kristallisationsneigung der Aminsalze mit zunehmender Konzentration, insbesondere bei hohen Protonierungsgraden, und andererseits durch die zunehmende gegenseitige Löslichkeit der Phasen ineinander, insbesondere bei niedrigen Protonierungsgraden.

Der Protonierungsgrad gibt das Verhältnis von Säureäquivalenten zu Aminäquivalenten wieder.

Nach unten wird dieser Bereich wirtschaftlich begrenzt durch den abnehmenden Säuregehalt und damit die quantitative Abnahme der Trennleistung, d.h. bei hervorragender qualitativer Trennleistung und technisch problemlos, ist mit sinkender Molarität ein zunehmend größeres Volumen an wäßriger Phase erforderlich für die Trennung einer gegebenen Aminmenge.

Gemäß einer Variante des erfindungsgemäßen Verfahrens erfolgt die Einspeisung des Ausgangspolyamingemisches (A) aus dem Vorratsbehälter (1) durch Vermischen mit dem Strom (B), gebildet aus dem Mengenstrom (E) der ersten Destillationsstufe (7.1), bestehend aus hydrophobem Lösungsmittel und gegebenenfalls Hilfsamin, und gegebenenfalls einem Teilstrom von (S).

Im allgemeinen liegt der Gehalt an Arylamin, bestehend aus Ausgangspolyamin und Hilfsamin in Mengenstrom (B) nach Zugabe von (A) bei 10 - 80 Gew.-%, vorzugsweise bei 15 - 60 Gew.-%.

Der Gehalt an Ausgangspolyamin in Mengenstrom (B) nach Zugabe von (A) liegt im allgemeinen bei 5 - 60 Gew.-%, vorzugsweise bei 10 - 40 Gew.-%.

Im Extraktor (3) wird der mit (A) beaufschlagte Mengenstrom (B) der wäßrigen Phase (C) entgegengeführt.

Im allgemeinen besteht der Mengenstrom (C) aus Wasser, einer starken Protonsäure, Hilfsamin und gegebenenfalls Polyamin.

Die Säure liegt in Form ihrer in Wasser gelösten Salze mit Hilfsamin und gegebenenfalls mit Polyamin vor. Die Summe der Aminogruppen von Hilfsamin und gegebenenfalls Polyamin liegen in (C) stets im stöchiometrischen Verhältnis oder im Überschuß vor, bezogen auf die Säure.

Der Protonierungsgrad beträgt in (C) im allgemeinen 40 - 100 %, für das vorzugsweise als Hilfsamin verwendete Anilin liegt er vorzugsweise bei 60 - 100 %.

Die für die jeweilige Ausführungsform des erfindungsgemäßen Verfahrens wohldefinierte und in engen Grenzen gemessene und geregelte Molarität des Mengenstrom (C) wird je nach der der jeweiligen Ausführungsform zugrunde liegenden - hier produktbezogenen - Trennaufgabe in einem weiten Bereich gezielt variiert.

Im allgemeinen hat die der Extraktionsstufe (3) des erfindungsgemäßen Verfahrens zugeführte wäßrige Phase (C) eine Molarität zwischen 0.5 und 4,5.

In der vorzugsweise mehrstufig betriebenen Extraktionsstufe (3) wird der mit (A) beaufschlagte Mengenstrom (B) und die wäßrige Phase (C) unter inniger Durchmischung einander entgegengeführt.

Bei diesem Vorgang findet in der Regel ein Übergang von Polyarylamin von der organischen Phase (B) in die wäßrige Phase statt, gegebenenfalls im Austausch gegen Hilfsamin in entgegengesetzter Richtung.

In der die Extraktionsstufe (3) verlassenden wäßrigen Phase (H) liegt die Säure als wäßrige Lösung ihrer Ammoniumsalze mit Polyamin und gegebenenfalls Hilfsamin vor, die in der Regel freies, d.h. nicht salzartig gebundenes Polyamin und gegebenenfalls freies, d.h. nicht salzartig gebundenes Hilfsamin gelöst enthält.

Das zusammen mit der organischen Phase (B) in den Extraktor (3) eingebrachte Ausgangspolyamin (A) verteilt sich auf die den Extraktor verlassende wäßrige - Phase (H) und die den Extraktor (3) verlassende organische Phase (D) (quantitative Fraktionierung).

Die mengenmäßige Aufteilung der einzelnen Komponenten des Ausgangspolyamingemisches auf die resultierende wäßrige Phase (H) und die resultierende organische Phase (D) erfolgt unter den Bedingungen des erfindungsgemäßen Verfahrens mit einer überraschend hohen Selektivität, so daß die resultierenden Produktfraktionen eine andere, unter Umständen stark von der des Ausgangspolyamingemisches abweichende Zusammensetzung aufweisen (qualitative Fraktionierung).

Beispielsweise ausgehend von den bevorzugt eingesetzten Anilinformaldehydkondensationsprodukten wurde gefunden, daß von einer in zwei oder mehreren isomeren Formen im Ausgangsgemisch enthaltenen Polyaminkomponente in der Regel die ortho-isomere(n) Form(en) in der die Trennstufe (3) verlassenden organischen Phase (D) relativ angereichert ist(sind); beispielsweise 2,4'-Diamino-diphenylmethan relativ zu 4,4'-Diamino-diphenylmethan. Umgekehrt ist die resultierende wäßrige Phase (H) relativ verarmt an dem 2,4-'Isomeren, während das 4,4'-Isomere relativ angereichert ist.

Sind mehrere "ortho-Isomere" im Ausgangspolyamin vorhanden, z.B. 2,2'-und 2,4'-Diamino-diphenylmethan, dann ist das "ortho-reichere" 2,2'-Isomere in der organischen Phase (D) gegenüber dem "ortho-ärmeren" 2,4'-Isomeren stärker angereichert, welchletzteres seinerseits gegenüber dem "noch ortho-ärmeren" 4,4'-Isomeren relativ angereichert ist.

Der zuerst bei den Anilinformaldehydkondensationsprodukten der Diamino-diphenylmethanreihe gefundene An- und Abreicherungseffekt wurde rein empirisch-deskriptiv mit dem Kriterium der ortho- und para- Substitution verbunden. Die davon abgeleitete Charakterisierung der Verfahrensprodukte als "orthoreich" und "orthoarm" ist dabei relativ und wurde durch den Begriff "ortho-Substitutionsgrad" ausgedrückt.

Als "ortho-Substitutionsgrad" wird dabei das Verhältnis der orthoständigen Aminogruppen-Methylengruppenrelationen, zur Gesamtzahl aller Aminogruppenrelationen definiert. Mit diesem Begriff lassen sich praktisch alle Isomerentrennungen bei den Polyaminen erfassen, die aus Arylaminen, auch substituierten, mit Carbonylverbindungen in wäßrig saurem Medium hergestellt werden.

Übertaschenderweise wurde nun der gleiche An- und Abreicherungseffekt - geordnet nach ortho-Substitutionsgrad - auch für die gut charakterisierten und analytisch erfaßbaren isomeren Dreikernverbindungen aus der Anilin-Formaldehydkondensation gefunden.

Analoges gilt für die Trennung der Isomeren von Kondensationsprodukten aus Formaldehyd mit Anilin und Diaminoarylverbindungen wie Phenylendiamin oder alkylsubstituierten Phenylendiaminen.

Die bisher erwähnten Polyamingemische weisen, bedingt durch ihre Herstellung, Aminogruppen auf, die praktisch nur orthoständig und/oder paraständig zu Methylengruppen sind. Dabei werden innerhalb einer Gruppe isomerer Verbindungen in der Regel diejenigen mit dem höheren ortho-Substitutionsgrad gegenüber den Isomeren mit einem geringeren ortho-Substitutionsgrad bei der Fraktionierung in der organischen Phase (D) angereichert.

Polyamingemische insbesondere der Diphenylmethanreihe einschließlich der jeweiligen höherkernigen Homologen, die nach anderen Verfahren hergestellt werden, beispielsweise durch Nitrierung von Diphenylmethan oder Methyldiphenylmethanen und anschließende Reduktion weisen außer ortho- und paraständigen Aminogruppen herstellungsbedingt auch andere Aminogruppen-Methylengruppenrelationen auf. Für diese Polyamingemische ist das erfindungsgemäße Verfahren genauso wirksam.

Beispielsweise läßt sich aus einem Gemisch von 2- und 4-Methyldiphenylmethan durch Nitrierung und anschließende Reduktion ein Polyamingemisch herstellen, welches in der Hauptsache ein Isomerengemisch darstellt aus

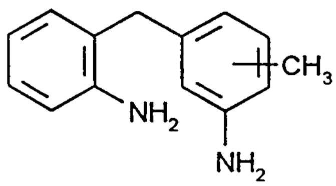

und

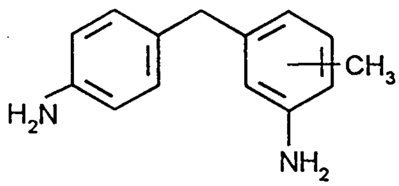

Bei der Fraktionierung solcher Gemische mit Hilfe des erfindungsgemäßen Verfahrens werden die 3.2'-Amino-Isomeren in der organischen Phase (D) gegenüber den 3,4'-Amino-Isomeren angereichert.

Das Kriterium "orthoreich" und "orthoarm" oder der ortho-Substitutionsgrad erfaßt in diesen Polyamingemischen nicht mehr alle Isomeren und ist daher sinngemäß anzuwenden, indem anstelle der Begriffe "orthoständig" und "paraständig" eine Einteilung der Isomeren vorgenommen wird in solche mit kleinerem (= ortho-) und solche mit größerem (= para-) räumlichen Abstand der - in der Regel an unterschiedlichen Sechsringen befindlichen - Aminogruppen zur Methylenbrücke bzw. der Aminogruppen zueinander.

Eine weitere Klasse aromatischer Polyamingemische, die sich mit Hilfe des erfindungsgemäßen Verfahrens sehr wirksam fraktionieren lassen, stellen die Polyamine des Triphenylmethans und seiner höherkernigen Homologen, vorzugsweise Benzylhomologen dar, wie sie z.B. durch Nitrierung und anschließende Reduktion der entsprechenden Kohlenwasserstoffgemische hergestellt werden.

Bei der Fraktionierung technischer Polyamingemische der zuletzt genannten Substanzklassen

I. Mischkondensationsprodukte von Mono- und Diaminoarylverbindungen mit Formaldehyd bzw. allgemeinen Carbonylverbindungen,

II. Polyamingemische aus Verfahren durch Nitrierung und anschließende Reduktion von Diphenylmethan und vorzugsweise substituierten insbesondere alkylsubstituierten Diphenylmethanen und den jeweiligen Homologen und

III. Polyamingemische aus Verfahren durch Nitrierung und anschließende Reduktion von Triphenylmethan und vorzugsweise substituierten insbesondere alkylsubstituierten Triphenylmethanen und den jeweiligen höherkernigen Benzylhomologen

wurde zusätzlich zur reinen Isomerentrennung eine weitere übertaschende Selektivität gefunden.

Polyamingemische der genannten Substanzklasse I bis III enthalten oder können enthalten Komponenten, bei denen wenigstens ein Arylkern pro Molekül mehr als eine, in der Regel zwei Aminogruppen trägt. Diese Komponenten können die bevorzugten Bestandteile des Polyamingemisches sein, ohne daß sie verfahrensbedingt mengenmäßig die Hauptprodukte sein müssen.

Zur besseren Charakterisierung solcher Komponenten wird der Begriff "Aminosubstitutionsgrad" verwendet, mit dem in erster Linie die Anzahl der Aminogruppen einer Komponente im Verhältnis zur Anzahl der Arylkerne gekennzeichnet wird.

Für Anilin und seine Kondensationsprodukte mit Formaldehyd ist dieser Ausdruck stets 1,0 , für Phenylendiamin und seine Kondensationsprodukte stets 2,0. Für reine Mischkondensate ergeben sich für die Diphenylmethanisomeren der Wert 1,5 und für die höherkernigen Homologen Werte zwischen > 1,0 und < 2,0. Bei statistischer Verwendung des Begriffes Aminosubstitutionsgrad zur Charakterisierung von Polyamingemischen ergeben sich ebenfalls Werte zwischen 1,0 und 2,0.

Bei der Fraktionierung von Polyamingemischen mit Komponenten mit einem Aminosubstitutionsgrad > 1,0 wurde nun gefunden, daß die Komponenten mit einem höheren Aminosubstitutionsgrad in der im eigentlichen Trennschritt

resultierenden wäßrigen Phase (H) relativ angereichert werden, und zwar um so stärker je größer der Aminosubstitutionsgrad ist.

Unabhängig davon ist auch hier die Trennung nach ortho-Substitutionsgrad wirksam.

Somit eröffnet das erfindungsgemäße Verfahren auch für diese Substanzklasse neue Wege, die Herstellungsform der Rohstoffe (Aminstufe) und die Verwendungsform der Endprodukte (Isocyanatstufe) durch Fraktionierung und/oder Anreicherung auf der Aminstufe und separate Weiterverarbeitung der Fraktionen zu entkoppeln, so daß eine getrennte Optimierung beider Stufen erleichtert wird bis hin zur Gewinnung völlig neuer Isocyanatgemische oder erst möglich wird wo bislang geeignete Verfahren und Methoden fehlten oder wenig praktikabel sind.

Ergänzt werden diese "Leistungen" durch ein weiteres Selektivitätskriterium, welches bei der Fraktionierung technischer Polyamingemische, insbesondere solcher mit höherkernigen Homologen, gefunden wurde und die "Kernigkeit" der Polyamingemische betrifft.

Mit dem Begriff "Kernigkeit" wird primär die Anzahl der Aryleinheiten einer Komponente eines aromatischen Polyamingemisches ausgedrückt. Im weiteren Sinne wird der Begriff der Kernigkeit dafür verwendet, um für ein aus zahlreichen Komponenten, mit einer individuellen exakten Kernigkeit, bestehendes Polyamingemisch statistisch eine Kernigkeit des Gesamtgemisches ausdrücken.

Besonders überraschenderweise wurde nun bei der Fraktionierung von Polyamingemischen mit höherkernigen Anteilen, insbesondere bei der Fraktionierung technischer Gemische von Anilin-Formaldehydkondensaten, gefunden, daß sich die höherkernigen Komponenten in der die Fraktionierungsstufe verlassenden organischen Phase gezielt sowohl relativ anreichern als auch relativ abreichern lassen, in Abhängigkeit von der Molarität der wäßrigen Phase in der Extraktionsstufe (3).

Eine hohe Molarität der wäßrigen Phase in (3) innerhalb des angegebenen Molaritätsbereichs führt zu einer relativen Abreichetung höherkerniger Komponenten in der organischen Phase (D) und dementsprechend zu einer relativen Anreicherung in der wäßrigen Phase (H).

Eine niedrige Molarität der wäßrigen Phase (C) innerhalb des angegebenen Molaritätsbereichs führt zu einer relativen Anreichetung höherkerniger Komponenten in der organischen Phase (D).

Der überraschende Befund kann dahingehend erweitert und präzisiert werden, daß die relative An- und Abreicherung auch innerhalb der höherkernigen Homologen untereinander stattfindet. Werden beispielweise in einem technischen Gemisch des Diamino-diphenylmethans in der einen Fraktion die Dreikernkomponenten gegenüber den Zweikernkomponenten relativ an- oder abgereichert, wird auch eine relative An- oder Abreicherung von Vierkernkomponenten gegenüber Dreikernkomponenten, d.h. eine noch stärkere relative An- oder Abreicherung, gefunden, desgleichen von Fünfkernkomponenten gegenüber Vierkernkomponenten u.s.w.

Daraus und aus der gleichzeitig und stets im Sinne einer relativen Verstärkung des "ortho-Substitutionsgrades" in der organischen Phase (D) ablaufenden Isomerentrennung und aus der Möglichkeit, mit einzelnen Produktfraktionen die erfindungsgemäße Trennung, gegebenenfalls mit veränderten Verfahrensparametern zu wiederholen, ergeben sich zahlreiche Möglichkeiten, ausgehend von bekannten und gut zugänglichen Polyamingemischen über das erfindungsgemäße Verfahren zu weniger gut zugänglichen oder völlig neuen, weil nach dem Stand der Technik bislang unzugänglichen, Polyaminen und damit Polyisocyanaten zu gelangen. Das gilt besonders für Produkte, der Diamino- und Diisocyanato-diphenylmethanreihe und ganz besonders für Polyamin- und Polyisocyanatgemische mit einem extrem hohen Anteil an höherkernigen Komponenten.

Die An- bzw. Abreicherung wird in der Regel effektiver mit steigendem Protonierungsgrad in der wäßrigen Phase der Trennstufe.

Darüber hinaus erweist sich das erfindungsgemäße Verfahren als von allgemeiner Wirksamkeit auch auf andere strukturähnliche Polyamine.

So können beispielsweise in den bereits erwähnten Polyamingemischen, die durch Nitrierung von Di- und Polyarylmethanen und anschließender Reduktion gewonnen werden, auch Monoaminopolyarylmethanverbindungen oder Komponenten enthalten sein, bei denen eine oder mehrere Methylengruppen durch Nebenreaktionen in Keto- und/oder Hydroxymethylengruppen und damit in unerwünschte Nebenprodukte umgewandelt worden sind.

Bei der Kondensation von Arylaminen mit Carbonylverbindungen können zahlreiche unvollständig umgelagerte Zwischenverbindungen und Nebenprodukte auftreten.

Die meisten dieser Verbindungen unterliegen in der Regel bei der Fraktionierung der sie enthaltenden Polyamingemische einer Anreicherung in einer der resultierenden Fraktionen, so daß der Effekt zur Abtrennung und Fraktionierung genutzt werden kann.

Gegebenenfalls können derartige Produkte auf diesem Wege an- oder abgereichert werden oder als gezielt hergestellte Polyamingemische, wie z.B. Polyaminobenzophenone oder Aminobenzylarylamingemische ihrerseits fraktioniert werden.

Die die Extraktionsstufe (3) verlassende, organische Phase (D) enthält unter anderem noch geringe Mengen an Säure, im allgemeinen und in Abhängigkeit von den Verfahrensparametern in der Extraktionsstufe (3) zwischen 0,01 und 0,5 Gew.-%, die vorteilhaft vor der destillativen Aufarbeitung des Mengenstromes (D) entfernt werden.

Im einfachsten Fall geschieht dies durch Neutralisation mit überschüssigen, verdünnten wäßrigen Basen, beispielsweise verdünnter Natronlauge. Bevorzugt wird jedoch das Herauswaschen der Säure bzw. ihrer Aminsalze aus der organischen Phase mit Wasser, so daß gegebenenfalls nur noch verbleibende Spuren durch Kontakt mit verdünnter Natronlauge oder mit Hilfe eines Ionentauschers entfernt werden.

Das eingesetzte Waschwasser wird unter Einschaltung eines Wasserverdampfers dem wäßrigen Säurekreislauf entzogen und diesem nach Durchlaufen der Waschstufe(n) mitsamt der Säure an verfahrensmäßig geeigneter Stelle wieder zugesetzt.

Die organische Phase (D) bzw. (D') wird, gegebenenfalls nach Durchlaufen der Säurewaschstufe (7.0), in die mindestens zweistufige Destillationsstufe (7.1), (7.2) überführt.

In der ersten Destillationsstufe (7.1) wird ein Destillat (E) abgetrennt, welches die Hauptmenge, vorzugsweise nahezu die Gesamtmenge des in (D) bzw. (D') enthaltenen, hydrophoben Lösungsmittels neben einem Teil des in ihr enthaltenen Hilfsamins umfaßt.

In der letzten Destillationsstufe (7.2) wird das verbleibende Hilfsamin, gegebenenfalls neben der Restmenge des hydrophoben Lösungsmittels, als Destillat (F) von dem als Destillationssumpf anfallenden und im Verfahrensprodukttank (10) gesammelten ersten Teilprodukt (G) abgetrennt.

Das entsprechende zweite Verfahrensprodukt befindet sich in der die Extraktionsstufe (3) verlassenden wäßrigen Phase (H).

In einer mehrstufig wirkenden, vorzugsweise bei 80 - 110° C betriebenen Extraktionsstufe (5) wird aus der wäßrigen Phase (H), gegebenenfalls nach Zugabe von Wasser zur Absenkung der Molarität und gegebenenfalls nach Zugabe von Hilfsamin zur Absenkung des Protonierungsgrades, das zweite Teilprodukt im Austausch gegen Hilfsamin extrahiert und dabei in die organische Phase (L) überführt.

Die Molarität der in (5) eingesetzten wäßrigen Phase ist vorzugsweise < 2,5.

Der Protonierungsgrad der in (5) eingesetzten wäßrigen Phase ist vorzugsweise < 60 %.

Als Extraktionsmittel (J) dient ein Gemisch aus hydrophobem Lösungsmittel und Hilfsamin, welches im wesentlichen aus der die nachfolgende Extraktionsstufe (6) verlassenden organischen Phase (R), gegebenenfalls Destillat (M) und in der Regel einer Teilmenge des Mengenstromes (S) gebildet wird.

Das Gewichtsverhältnis von Hilfsamin zu Lösungsmittel liegt in (J) im allgemeinen zwischen 0,5 : 1 und 3 : 1, vorzugsweise zwischen 1 : 1 und 2 : 1.

Das Gewichtsverhältnis von Extraktionsmittel (J) zu wäßriger Phase liegt im allgemeinen zwischen 0,3 : 1 und 3 : 1, vorzugsweise zwischen von 0.7 : 1 und 2 : 1.

Die in (5) resultierende, organische Phase (L) bzw. (L') wird, gegebenenfalls nach Durchlaufen der Waschstufe (8.0) und/oder gegebenenfalls nach Entfernen von Säurespuren mit verdünnter Natronlauge, den Destillationsstufen (8.1) und (8.2) zugeführt.

In der Destillationsstufe (8.1), (8.2) erfolgt die destillative Abtrennung des Destillationsrückstandes (O), der als zweites Teilprodukt in den Verfahrensprodukttank (11) gesammelt wird.

Die Aufarbeitungsstufe (8) besteht im allgemeinen aus einer wenigstens zweistufigen Mehrstufendestillation (8.1) und (8.2), in deren Stufe (8.1) ein gegenüber dem Zulaufprodukt (L) bzw. (L') von Polyamin befreites und an Hilfsamin stark verarmtes hydrophobes Lösungsmittel als Destillat (M) gewonnen wird.

Der Gehalt von (M) an Hilfsamin beträgt im allgemeinen < 30 %, vorzugsweise < 20 %; gegebenenfalls wird in (8.1) ein von Hilfsamin befreites hydrophobes Lösungsmittel als Destillat gewonnen.

Das Destillat (M) wird, zumindest teilweise gegebenenfalls auch nach Zugabe von einer Teilmenge von (E) aus der Destillationsstufe (7.1), eingesetzt in der Extraktionsstufe (6) als organische Phase (P) zur Gegenstromextraktion von wenigstens einer Teilmenge der in der Extraktionsstufe (5) resultierenden wäßrigen Phase (K), welche der Extraktionsstufe (6) direkt und/oder indirekt über eine zwischengeschaltete Destillationsstufe (9) zumindest teilweise zugeführt wird.

Bei dem in der Extraktionsstufe (6) ablaufenden Extraktionsvorgang wird der wäßrigen Phase Hilfsamin und gegebenenfalls noch vorhandenes Polyamin entzogen, so daß die resultierende wäßrige Phase (Q) stets einen höheren Protonierungsgrad aufweist als die wäßrige Phase (K) aus der Extraktionsstufe (5).

Der Protonierungsgrad von (Q) beträgt im allgemeinen > 50 %, vorzugsweise > 60 % und kann bei Verwendung von reinen hydrophobem Lösungsmittel als (P) bis zu 100 % betragen.

Es ist ein Kennzeichen des erfindungsgemäßen Verfahrens, daß die wiederverwendete wäßrige Phase (C) einen höheren Protonierungsgrad aufweist als die die Extraktionsstufe (5) verlassende wäßrige Phase (K).

Für Protonierungsgrade, die zwar höher als derjenige von (K) aber < 100 % betragen, kann das erreicht werden, indem entweder die gesamte wäßrige Phase die Extraktionsstufe (6) durchläuft oder nur eine entsprechende Teilmenge, die gegebenenfalls mit einem Protonierungsgrad von 100 % durch Vermischen mit dem Rest den gegenüber (K) erhöhten Protonierungsgrad von (C) ergibt.

Ein Protonierungsgrad von praktisch 100 % in der wäßrigen Phase (C) kann nur erreicht werden, wenn die gesamte wäßrige Phase wenigstens einmal über die Extraktionsstufe (6) läuft und dabei mit praktisch reinen hydrophobem Lösungsmittel extrahiert wird.

Da die wäßrige Phase (C) erfindungsgemäß einen höheren Protonierungsgrad aufweist als den in der wäßrigen Eingangsphase zur Extraktionsstufe (5) bevorzugten Protonierungsgrad, kann es erforderlich und vorteilhaft sein, durch Zugabe von Hilfsamin und/oder Polyamin an geeigneter Stelle einen solchen bevorzugten Protonierungsgrad in der wäßrigen Eingangsphase zur Extraktionsstufe (5) einzustellen.

Geeignete Stellen für die Aminzufuhr sind die wäßrige Phase unmittelbar vor Eintritt in die Extraktionsstufe (5) unter Verwendung von Hilfsamin oder an vorgelagerter Stelle, vorzugsweise vor Eintritt der wäßrigen Phase in die Extraktionsstufe (3) beispielsweise durch direkte Zugabe oder über eine vorgeschaltete Extrakionsstufe (2).

Ein weiterer wichtiger Parameter der zurückgeführten und wiederverwendeten wäßrigen Phase (C) ist die Molarität. Erfindungsgemäß kann die Molarität von (C) in einem weiten Bereich variiert werden in Abhängigkeit von der jeweiligen Trennaufgabe.

Grundsätzlich kann die in Verfahrensstufe (3) resultierende wäßrige Phase (H) direkt oder gegebenenfalls über eine Zwischenstufe (4) der Extraktionsstufe (5) zugeführt werden. Da aber der obere, für bestimmte Trennaufgaben durchaus bevorzugte Molaritätsbereich der Extraktionsstufe (3) oberhalb des bevorzugten Molaritätsbereichs der Extraktionsstufe (5) liegt, ist es eine bevorzugte Ausführungsform des erfindungsgemäßen Verfahrens, die Molaritäten in den verschiedenen Extraktionsstufen dadurch zu entkoppeln, daß dem in sich geschlossenen System der wäßrigen Phase gegebenenfalls an geeigneter Stelle destillativ Wasser entzogen und an anderer geeigneter Stelle wieder zugesetzt wird.

Mit Hilfe einer Wasserdestillationsstufe (9) wird der wäßrigen, die Säure enthaltende Phase, oder einem Teilstrom der wäßrigen Phase, vorzugsweise nach Verlassen der Extraktionsstufe (5) und vor der Wiederverwendung am Prozessbeginn Wasser entzogen, welches vor Eintritt der wäßrigen Phase in die Extraktionsstufe (5) insgesamt (Y) oder in Teilmengen an einer oder mehreren Stellen wieder zugesetzt wird.

Ob der Entzug von Wasser in (9) vor oder nach Durchlaufen der Extraktionsstufe (6) erfolgt, ist für die erfindungsgemäße Durchführung ohne Belang.

Das in (9) entzogene Wasser (X) kann gleichzeitig zum Betreiben der Waschstufen (7.0) und/oder (8.0) genutzt werden.

Die Wassermenge (X) kann auch überwiegend oder ausschließlich zum Betreiben der Waschstufen (7.0) und/oder (8.0) entzogen werden.

Mit dieser ersten Variante des erfindungsgemäßen Verfahrens lassen sich beträchtliche Trennleistungen bei der Fraktionierung von Polyamingemischen erzielen und zahlreiche Trennprobleme zufriedenstellend lösen.

Insbesondere in der ersten Polyaminfraktion (G) kann die relative Anreicherung der in dieser Fraktion bevorzugt enthaltenen Komponenten gezielt variiert und maximiert werden.

Der in der zweiten Polyaminfraktion (O) verbleibende Anteil dieser Komponenten kann jedoch gemaß dieser ersten Variante nicht in gleicher Weise minimiert werden, sondern variabel nur bis zu einem Gehalt relativ abgereichert werden, dessen untere Grenze abhängt von dem für die jeweiligen Verfahrensparameter charakteristischen Verteilungsgleichgewichte der Polyaminkomponenten von (A) zwischen der organischen Phase (B) beim Eintritt in den Extraktor (3) und der wäßrigen Phase (H) beim Verlassen des Extraktors (3).

Durch Zumischen von Anteilen der zweiten Polyaminfraktion (O) vorzugsweise als Teilstrom von (L) zum Ausgangsarylamin (A) kann dessen Gehalt an Komponenten von (G) relativ gesenkt und damit über das Verteilungsgleichgewicht die gemäß erster Variante erreichbare Untergrenze in diesen Komponenten im zweiten Teilprodukt (O) verschoben werden. Die damit erzielte nur graduelle Verbesserung der Trennleistung mag für bestimmte Anwendungen ausreichend sein, geht in der Regel aber zu Lasten des Durchsatzes an (A).

Vorteilhafter und als Ausführungsform bevorzugt ist eine zweite Variante des erfindungsgemäßen Verfahrens, bei der sich zusätzlich auch in der zweiten Polyaminfraktion (O) die relative Anreicherung der in dieser Fraktion bevorzugt enthaltenen Komponenten weitgehend unabhängig von der ersten Produktfraktion, gezielt variieren läßt, indem die in der Extraktionsstufe (3) anfallende wäßrige Phase (H) oder zumindest eine Teilmenge derselben in einer zwischengeschalteten, nach dem Gegenstromprinzip arbeitenden Extraktionsstufe (4) mit einer organischen Phase (T) extrahiert wird.

Die organische Phase (T) besteht im allgemeinen neben hydrophobem Lösungsmittel aus Hilfsamin und/oder Polyamin, letzteres vorzugsweise mit der Zusammensetzung des zweiten Verfahrensteilproduktes (O).

Bei der Verwendung einer organischen Phase (T) ohne Polyamin resultiert in der die Extraktionsstufe (4) verlassenden wäßrigen Phase (V) eine Polyaminfraktion, in welcher die relative Anreicherung der in dieser Phase bevorzugt enthaltenen Komponenten über die in der wäßrigen Phase (H) erzielte Anreicherung hinaus gezielt erhöht und maximiert werden kann, auf Kosten der Polyaminkonzentration in der wäßrigen Phase (V).

Polyamin als Bestandteil der organischen Phase (T) bewirkt, daß die die Verfahrensstufe (4) verlassenden Phasen (U) und (V) eine höhere und damit für die Durchführung des erfindungsgemäßen Verfahrens energetisch vorteilhaftere Polyaminkonzentration aufweisen, als bei der Verwendung einer organischen Phase (T) ohne Polyamin.

Durch die bevorzugte Verwendung eines Polyamins mit der Zusammensetzung des zweiten Teilprodukts (O) als

Bestandteil der organischen Phase (T) kann auch auf einem höheren und damit vorteilhaften Konzentrationsniveau infolge Gleichgewichtseinstellung mit Selbstverstärkung des Trenneffektes die relative Anreicherung der in der die Trennstufe (4) verlassenden wäßrigen Phase (V) bevorzugt enthaltenen Polyaminkomponenten und damit der zweiten Polyaminfraktion (O) variiert und maximiert werden.

Im einfachsten und allgemeinen Fall wird die organische Phase (T) gebildet aus zumindest einem Teilstrom von Mengenstrom (E) und gegebenenfalls weiterem Hilfsamin aus Mengenstrom (S). Vorzugsweise wird Mengenstrom (T), gegebenenfalls aus einer Teilmenge von (E), gegebenenfalls einer Teilmenge von (S) und einer Teilmenge des Mengenstromes (L), enthaltend das Polyamingemisch (O), gebildet.

Gegebenenfalls kann vollständig auf die Zuspeisung von hydrophobem Lösungsmittel und Hilfsamin aus anderen Quellen verzichtet werden, so daß die organische Phase (T) ausschließlich aus einer Teilmenge von (L) besteht.

Diese Ausführungsform des erfindungsgemäßen Verfahrens ist bezüglich Trennleistung und Energiebilanz besonders vorteilhaft und daher besonders bevorzugt, wenn sie angewendet werden kann.

Die Molarität der in der zwischengeschalteten Extraktionsstufe (4) eingesetzten wäßrigen Phase liegt im allgemeinen auf gleicher Höhe wie in der die Extraktionsstufe (3) verlassenden wäßrigen Phase (H). Es ist jedoch möglich durch Zugabe von Wasser gegebenenfalls auch von Hilfsamin zur wäßrigen Phase sowohl die Molarität als auch den Protonierungsgrad zur Verbesserung der verfahrensgemäßen Trennleistung zu verändern.

Grundsätzlich ist es auch möglich, die Molarität von der in (4) eingesetzten wäßrigen Phase durch destillativen Entzug von Wasser zu erhöhen.

Die in Stufe (4) resultierende organische Phase (U) wird der in Extraktionsstufe (3) eingesetzten organischen Phase (B) zugesetzt.

Die in Stufe (4) resultierende wäßrige Phase (V) wird, gegebenenfalls mit dem Rest von (H), der Extraktionsstufe (5) zugeführt.

Für diejenige Menge an hydrophobem Lösungsmittel, welche gegebenenfalls zur Bildung des Extraktionsmittels (T) dem Lösungsmittelkreislauf des zweiten Verfahrensproduktes (O) durch Abzweigen eines Teilstromes von (L) entzogen wird, erfolgt ein Mengenausgleich vorzugsweise durch Zugabe einer Teilmenge von (E) bei der Bildung der organischen Phasen (P) und/oder (J).

Mit der zweiten Variante des erfindungsgemäßen Verfahrens lassen sich die relative Anreicherung in beiden resultierenden Polyaminfraktionen gezielt variieren und maximieren. Neben dieser in qualitativer Hinsicht großen Vielseitigkeit und Leistungsfähigkeit bietet die zweite Verfahrensvariante zumindest für die zweite Polyaminfraktion (O) auch eine energetisch günstige Ausführungsform.

Der mit Gewinnung der ersten Polyaminfraktion (G) verbundene Aufwand steigt dagegen relativ um so stärker, je geringer der mengenmäßige Anteil von (G), bezogen auf eingesetztes Polyamingemisch (A) ist, weil der verbleibende Gehalt an Polyamin (G) in der destillativ aufzuarbeitenden organischen Phase (D) entsprechend immer kleiner wird.

Der Effekt kommt besonders dann zum Tragen, wenn die mit (G) abgetrennten Komponenten im Ausgangsgemisch (A) nur in geringer Konzentration enthalten sind und/oder relativ hoch in der Fraktion (G) angereichert werden, z.B. bei der erfindungsgemäßen Auftrennung von Polyamingemischen der Diphenylmethanreihe.

Eine teilweise Einbringung von Ausgangspolyamin (A) in die Trennstufe (3) über die zugeführte wäßrige Phase bringt in der Regel eine Erhöhung der Polyaminkonzentration in (D) und damit ernergetische Entlastung. Bei der erfindungsgemäßen Durchführung des Verfahrens ist diese Entlastung aber infolge der Gleichgewichtseinstellung zwischen dem Polyamin in der zugeführten wäßrigen Phase und dem Polyamin in der organischen Phase (D) mit einer Verschlechterung des qualitativen Trennergebnisses im ersten Teilprodukt (G) verbunden, so daß von dieser Möglichkeit nur im untergeordneten Maße oder in Fällen mit entsprechend geringen Anforderungen an das Trennergebnis Gebrauch gemacht wird.

Eine in dieser Hinsicht verbesserte Ausführung stellt die dritte Variante des erfindungsgemäßen Verfahrens dar. Ausgehend von der ersten Variante wird diese dahingehend erweitert, daß die die Verfahrensstufe (3) verlassende, das erste Teilprodukt (G) in gegenüber der Konzentration von (A) in (B) verringerter Konzentration, enthaltende organische Phase (D) geteilt wird in einen Mengenstrom (D'), der weiterhin mit dem Ziel der Gewinnung der Polyaminfraktion (G) der Aufarbeitungsstufe (7) zugeführt wird, und in einen Mengenstrom (D").

Der Mengenstrom (D") wird in einer vorgelagerten Extraktionsstufe (2) mit zumindest einer Teilmenge, vorzugsweise mit der Gesamtmenge der zur Wiederverwendung zur Verfügung stehenden wäßrigen Phase (C) im Gegenstrom extrahiert.

Der dem Extraktor (2) zugeführte Mengenstrom (D") wird dabei so bemessen, daß bei der Umsetzung mit Mengenstrom (C) ein möglichst weitgehender, vorzugsweise praktisch quantitativer Übergang der in der organischen Phase (D") enthaltenen Polyamine in die den Extraktor (2) verlassende wäßrige Phase erfolgt, so daß eine möglichst weitgehend von Polyamin befreite organische Phase (Z) resultiert.

Ein erhöhter Protonierungsgrad in der in Stufe (2) eingesetzten wäßrigen Phase, resultierend aus der Verfahrensstufe (6) des erfindungsgemäßen Verfahrens, ebenso wie eine erhöhte Molarität in der in Stufe (2) eingesetzten wäßrigen Phase, resultierend aus Verfahrensstufe (9) des erfindungsgemäßen Verfahrens, begünstigen und erleichtern

den Übergang von Polyamin aus der organischen Phase (D") in die wäßrige Phase.

Der Restgehalt an Polyamin in der die Verfahrensstufe (2) verlassenden organischen Phase (Z) liegt im allgemeinen bei < 5 Gew.-%, vorzugsweise bei < 1 Gew.-%.

Im übrigen richtet sich der in (Z) zulässige Höchstgehalt an Amin und insbesondere der Gehalt an Polyamin nach den aus der jeweiligen Trennaufgabe resultierenden qualitativen Anforderungen an die Verfahrensprodukte, d.h. die Qualität der Trennung, im Falle der Variante 3 insbesondere an das Verfahrensteilprodukt (O). Die Einhaltung des für die Qualität von (O) relevanten Gehaltes an Polyamin wird im Rahmen der technischen Gegebenheiten unter Ausschöpfung des zur Verfügung stehenden Potentials an wäßriger Phase (C) über die Bemessung des Teilmengenstromes (D") kontrolliert.

In quantitativer Hinsicht kommt es dem Verfahren und insbesondere der vorgelagerten Verfahrensstufe (2) zustatten, daß das Verhältnis von (D") zu (D') besonders dann zu höheren Werten tendiert, d.h. daß (D") mengenmäßig größer wird, wenn das Mengenverhältnis der Polyaminfraktionen (G) zu (O) kleiner wird, weil (O) auf Kosten von (G) größer wird. Mit zunehmenden Anteil der zweiten Polyaminfraktion (O) erhöht sich die im Kreis geführte und damit in (2) zur Extraktion von (D") zur Verfügung stehende wäßrige Phase (C).

Die an Amin verarmte, insbesondere von Polyamin praktisch befreite, die Verfahrensstufe (2) verlassende organische Phase (Z) wird der organischen Phase (B) zugeschlagen und zusammen mit dieser als Lösungsmittel für Ausgangspolyamin (A) der Verfahrensstufe (3) zugeführt.

Die die Verfahrensstufe (2) verlassende wäßrige Phase enthält neben der eingesetzten wäßrigen Säure Polyamin, welches in seiner Zusammensetzung weitgehend dem in (D) abgetrennten und als Fraktion (G) isolierten Polyamin entspricht, und gegebenenfalls Hilfsamin.

Im einfachsten Falle wird die die Verfahrensstufe (2) verlassende wäßrige Phase direkt als Mengenstrom (C) der Verfahrensstufe (3) zugeführt; gegebenenfalls werden vorher weitere wäßrige Phase und/oder Wasser und/oder Hilfsamin zugemischt.

Auch die Zugabe einer begrenzten Menge von Ausgangspolyamin (A) zu der wäßrigen Phase ist gemaß Variante 3 möglich, ohne Einbuße an qualitativer Trennleistung (relativer Anreicherung) gegenüber Variante 1, dafür aber bei verbesserter quantitativer Leistung (größerer Wirtschaftlichkeit).

Bei einer weiteren vierten Variante wird die vorgelagerte Verfahrensstufe (2) und die sich ergebenden Vorteile kombiniert mit der als Variante 2 beschriebenen Ausführungsform des erfindungsgemäßen Verfahrens, die dadurch bezüglich des ersten Teilproduktes eine analoge Verbesserung erfährt wie bei Variante 3 beschrieben. Als eine zusätzliche Erleichterung und Vereinfachung für die Durchführung der Verfahrensstufe (2) erweist es sich, daß die Anforderungen an den verbleibenden Polyamingehalt der in (2) resultierenden organischen Phase (Z) weniger streng sind, da die nachteiligen Auswirkungen eines erhöhten Polyamingehaltes in (Z) auf die Qualität des zweiten Verfahrensteilproduktes (O) durch die nachgeschaltete Extraktionsstufe (4) kompensiert werden können.

Der Gehalt an Polyamin in der die Verfahrensstufe (2) verlassenden organischen Phase (Z) liegt daher im allgemeinen bei < 5 Gew.-%, vorzugsweise bei < 3 Gew.-%.

**Beispiel 1**

Ausgangspolyamingemisch (A) (2,600 kg/h) wird mit dem im wesentlichen aus Polyamingemisch, Anilin und Xylol bestehenden Mengenstrom (B) (7,835 kg/h) vermischt.

Mengenstrom (B) wird gebildet im wesentlichen aus Mengenstrom (U) (4,255 kg/h) und Mengenstrom (Z) (3,580 kg/h).

Die resultierende organische Phase (Mengenströme A+B) hat im Mittel folgende Zusammensetzung

| | |
|---|---|
| Mengenstrom (A)+(B) ( 10,435 kg/h ) | 43,4 % Polyarylamin |
| | 7,9 % Anilin |
| | 48,7 % Xylol |

und wird in einem mehrstufig wirkenden Extraktor (3) bei 90°-95°C dem Mengenstrom (C) entgegengeführt, der die folgende durchschnittliche Zusammensetzung hat:

| | |
|---|---|
| Mengenstrom (C) ( 28,650 kg/h ) | 4,4 % Polyarylamin |
| | 11,9 % Anilin |
| | 3,3 % Chlorwasserstoff |
| | 80,4 % Wasser. |

Die den Extraktor (3) verlassende organische Phase (D) hat folgende durchschnittliche Zusammensetzung:

| | |
|---|---|
| Mengenstrom (D) ( 10,650 kg/h ) | 21,5 % Polyarylamin |
| | 30,8 % Anilin |
| | 47,7 % Xylol. |

Mengenstrom (D) wird aufgeteilt in Teilmengenstrom (D"), welcher der Extraktionsstufe (2) zugeführt wird und den Mengenstrom (D') (4,870 kg/h).

Mengenstrom (D') wird im Extraktor (7.0) mit Wasser (ca. 0,5 - 1,0 kg/h) gewaschen. Das resultierende Waschwasser aus (7.0) [analog aus (8.0)] wird in Mengenstrom (Y) gesammelt.

Zur Sicherheit wird Mengenstrom (D') mit verdünnter Natronlauge gewaschen. Die wäßrige Phase wird als Abwasser entsorgt.

Von dem gewaschenen und von Säureresten befreiten Mengenstrom (D') wird in einer ersten Destillationsstufe (7.1) eine erste Destillatfraktion (E) abgetrennt. Der resultierende Destillationsrückstand wird in einer zweiten Destillationsstufe (7.2) in eine zweite Destillatfraktion (F), einer Teilmenge von (S), und einen Destillationsrückstand (G) aufgetrennt.

Der Destillationsrückstand von Destillationsstufe (7.2) besteht aus einer ersten Polyaminfraktion (G) mit 1,045 kg/h und wird in Tank (10) gesammelt.

Die den Extraktor (3) verlassende wäßrige Phase (H) hat folgende durchschnittliche Zusammensetzung

| | |
|---|---|
| Mengenstrom (H) ( 28,435 kg/h ) | 12,3 % Polyarylamin |
| | 3,4 % Anilin |
| | 3,3 % Chlorwasserstoff |
| | 81,0 % Wasser |

und wird in einem weiteren mehrstufig wirkenden Extraktor (4) bei 90°-95°C einer organischen Phase (T) entgegengeführt, die eine Teilmenge der organischen Phase (L) ist.

Mengenstrom (T) ( 5,150 kg/h )

Die in Verfahrensstufe (4) resultierende organische Phase (U) wird der Verfahrensstufe (3) zugeführt und geht in Mengenstrom (B) ein.

Mengenstrom (U) ( 4,255 kg/h )

Die im Extraktor (4) resultierende wäßrige Phase (V)

Mengenstrom (V) ( 29,330 kg/h )

wird in einem weiteren mehrstufig wirkenden Extraktor (5) bei 90-95°C dem Mengenstrom (J) entgegengeführt

| | |
|---|---|
| Mengenstrom (J) ( 13,070 kg/h ) | <0,1 % Polyarylamin |
| | 50,2 % Anilin |
| | 49,7 % Xylol. |

Die den Extraktor (5) verlassende organische Phase (L) fällt an mit der folgenden durchschnittlichen Zusammensetzung

| | |
|---|---|
| Mengenstrom (L) ( 14,400 kg/h ) | 16,8 % Polyarylamin |
| | 38,1 % Anilin |
| | 45,1 % Xylol |

und wird aufgeteilt in den Teilstrom, welcher als Mengenstrom (T) der Extraktionsstufe (4) zugeführt wird, und den Mengenstrom (L').

Mengenstrom (L') (9,250 kg/h) wird in einen Extraktor (8.0) mit Wasser (ca. 1,0 - 1,5 kg/h) gewaschen. Das resultierende Waschwasser wird im Mengenstrom (Y) gesammelt.

Zur Sicherheit wird Mengenstrom (L') mit verdünnter Natronlauge gewaschen. Die wäßrige Phase wird als Abwasser entsorgt.

Der gewaschene und von Säureresten befreiten Mengenstrom (L') wird anschließend in einer ersten Destillationsstufe (8.1) aufgetrennt in eine erste Destillatfraktion (M), die praktisch aus Xylol mit einen Anilingehalt von maximal 1

% besteht

Mengenstrom (M) ( 4,200 kg/h )

und in einen Destillationssumpf, welcher in einem zweiten Destillationsschritt aufgetrennt wird in eine zweite Destillatfraktion (N), einer praktisch aus Anilin bestehenden Teilmenge von (S),

Mengenstrom (N) ( 3,495 kg/h )

und in einen aus Polyamin bestehenden Destillationsrückstand (O).

Mengenstrom (O) ( 1,555 kg/h )

Die Polyaminfraktion (O) wird als zweites Teilprodukt in Tank (11) gesammelt.

Die Destillatfraktion (E) und Mengenstrom (S) werden der Extraktionsstufe (5) zugeführt und bilden zusammen mit Mengenstrom (R) die organische Eingangsphase (J).

Die den Extraktor (5) verlassende wäßrige Phase (K) hat folgende durchschnittliche Zusammensetzung

| | |
|---|---|
| Mengenstrom (K) ( 28,000 kg/h ) | 0,2 % Polyarylamin |
| | 14,2 % Anilin |
| | 3,3 % Chlorwasserstoff |
| | 82,3 % Wasser |

und wird in einem weiteren mehrstufig wirkenden Extraktor (6) bei 90°-95°C einer organischen Phase (P) entgegengeführt, (P) ist im vorliegenden Fall identisch nach Menge und Zusanmmensetzung mit der ersten Destillatfraktion (M) aus der Destillationsstufe (8.1).

Bei diesen Vorgang resultiert als organische Phase der Mengenstrom (R),

| | |
|---|---|
| Mengenstrom (R) ( 5,750 kg/h ) | <0,2 % Polyarylamin |
| | 27,4 % Anilin |
| | 72,6 % Xylol. |

Die in (6) resultierende wäßrige Phase (Q) hat folgende durchschnittliche Zusammensetzung:

| | |
|---|---|
| Mengenstrom (Q) ( 26,450 kg/h ) | 0,2 % Polyarylamin |
| | 9,2 % Anilin |
| | 3,5 % Chlorwasserstoff |
| | 87,1 % Wasser. |

In einer nachfolgenden Destillationsstufe (9) wird der wäßrigen Phase (Q) destillativ Wasser in Form des Mengenstrom (X) entzogen.

Mengenstrom (X) (ca. 1,5- 2,5 kg/h )

Das abdestillierte Wasser wird in Teilströmen in den Waschstufen (7.0) und (8.0) zum Waschen der organischen Phasen eingesetzt. Die resultierenden Waschwässer werden im Mengenstrom (Y) vereinigt.

Die in (9) resultierende konzentrierte wäßrige Phase (W) wird in der vorgelagerten Extraktionsstufe (2) bei 90°-95°C dem organischen Teilstrom (D") entgegengeführt. Bei diesen mehrstufig ablaufenden Vorgang resultiert eine organische Phase (Z) mit der folgenden Zusammensetzung:

| | |
|---|---|
| Mengenstrom (Z) ( 3,580 kg/h ) | <1 % Polyarylamin |
| | 21,8 % Anilin |
| | 77,1 % Xylol |

die zur Bildung von Mengenstrom (B) verwendet wird.

Die in der Extraktionsstufe (2) resultierende wäßrige Phase wird mit Mengenstrom (Y) vereinigt und als wäßrige Phase (C) der Extraktionsstufe (3) zugeführt.

In den Extraktionsstufen (2) bis (6) werden organische Phasen und wäßrige Phasen einander entgegengeführt.

Daraus resultieren geringe Gehalte an Chlorwasserstoff und Wasser in den organischen Phasen einerseits und geringe Gehalte an Xylol in den wäßrigen Phasen andererseits. Diese bleiben bei der Bilanzierung und Charakterisierung der Mengenströme unberücksichtigt.

Die über die Sicherheitsneutralisationsstufen bei der Wäsche mit Natronlauge dem System entzogenen Säure - und Wassermengen werden durch Zugabe von außen ersetzt.

| Polyarylamin GC: | A [Gew. - %] | G [Gew. - %] | O [Gew. - %] |
|---|---|---|---|
| 2,2' Diamino-diphenylmethan | <0,10 | <0,10 | ---- |
| 2,4'-Diamino-diphenylmethan | 0,72 | 1,80 | <0,10 |
| 4,4'-Diamino-diphenylmethan | 66,00 | 17,50 | 98,60 |
| N-Methyl-4,4'-diamino-diphenylmethan | 0,10 | 0,20 | ---- |
| Σ-Diamino-diphenylmethane | 66,90 | 19,50 | 98,60 |
| Σ-Mehrkernpolyamine | 33,10 | 80,50 | 1,30 |
| Mengenverteilung | 100 % | 40,2 % | 59,8 % |

**Patentansprüche**

1. Verfahren zur Fraktionierung und Reinigung von aromatischen Polyamingemischen der Diphenylmethanreihe, dadurch gekennzeichnet, daß man

   a) das Ausgangspolyamingemisch (A) in einem zweiphasigen System bestehend aus (i) einer hydrophoben Lösungsmittelphase (B), die im wesentlichen aus hydrophobem Lösungsmittel besteht, und (ii) einer wäßrigen Phase (C), bestehend im wesentlichen aus Wasser, einer starken Säure und zumindest teilweise in der Salzform vorliegendem Hilfsamin, welches in Wasser praktisch unlöslich ist und unter Normaldruck einen mindestens 20°C unter dem Siedepunkt der am niedrigsten siedenden Komponente des Ausgangsgemisches und mindestens 20°C oberhalb des Siedepunktes des Lösungsmittels liegenden Siedepunkt aufweist, unter Zuhilfenahme einer, nach dem Gegenstromprinzip arbeitenden Extraktionsstufe (3) unter Durchmischung der Phasen verteilt, indem man das Ausgangspolyamingemisch in die Extraktionsstufe (3) einbringt, und die die Extraktionsstufe verlassende organische Phase (D), zumindest teilweise, in einer mehrstufigen Destillation (7.1), (7.2) in eine erste, in der Extraktionsstufe (3) wiederverwendeten Fraktion (E), bestehend im wesentlichen aus hydrophobem Lösungsmittel, eine zweite Fraktion (F), bestehend im wesentlichen aus Hilfsamin und einem Destillationsrückstand (G), bestehend im wesentlichen aus einer ersten Polyaminfraktion, auftrennt und die die Extraktionsstufe (3) verlassende wäßrige Phase (H)

   b) in eine Extraktionsstufe (5) leitet,in welcher nach dem Prinzip der Gegenstromextraktion eine Extraktion der wäßrigen Phase mit einer aus hydrophobem Lösungsmittel und Hilfsamin bestehenden Lösungsmittelphase (J) erfolgt, wobei die an Polyamin verarmte wäßrige Phase (K) resultiert, und

   c) die in der Extraktionsstufe (5) anfallende organische Phase (L) zumindest teilweise, in einer mehrstufigen Destillation (8.1), (8.2) in eine erste Fraktion (M), bestehend im wesentlichen aus hydrophobem Lösungsmittel und eine zweite Fraktion (N) bestehend im wesentlichen aus Hilfsamin und einen Destillationsrückstand (O), bestehend im wesentlichen aus einer zweiten Polyaminfraktion, auftrennt und

   d) die in der Extraktionsstufe (5) anfallende wäßrige Phase (K) zumindest teilweise,

   e) in eine Extraktionsstufe (6) leitet und im Gegenstrom mit einer organischen Phase (P) extrahiert, bestehend im wesentlichen aus hydrophobem Lösungsmittel und höchstens soviel Hilfsamin, daß in (6) eine gegenüber

der eingesetzten wäßrigen Phase (K) an Arylamin verarmte wäßrige Phase (Q) resultiert, die man

f) als Mengenstrom (C) wiederverwendet, indem man Mengenstrom (C)

g) der Extraktionsstufe (3) zuführt und

g) die in der Extraktionsstufe (6) anfallende organische Phase (R) als Extraktionsmittelphase (J) der Extraktionsstufe (5) zuführt.

2. Verfahren gemäß Anspruch 1, dadurch gekennzeichnet, daß man

b) die in der Extraktionsstufe (3) anfallende wäßrige Phase (H) zumindest teilweise in einer nachgeschalteteten, nach dem Gegenstromprinzip arbeitenden Extraktionsstufe (4) extrahiert unter Verwendung einer organischen Phase (T) als Extraktionsmittel, bestehend neben hydrophobem Lösungsmittel aus Hilfsamin und Polyamin, letzteres eingebracht als Teilmenge des Mengenstromes (L), die in Verfahrensstufe (4) resultierende organische Phase (U) dem Mengenstrom (B) und damit der Extraktionsstufe (3) und die resultierende wäßrige Phase (V) der Extraktionsstufe (5) zuführt.

3. Verfahren gemäß Anspruch 1 oder 2, dadurch gekennzeichnet,daß man

j) eine Teilmenge (D") der die Extraktionsstufe (3) verlassenden organischen Phase (D) abtrennt und in einer vorgelagerten, vorzugsweise mehrstufigen, Extraktionsstufe (2) mit dem gesamten Mengenstrom (C) im Gegenstrom extrahiert, gegebenenfalls unter Zugabe von Hilfsamin, und den Teilmengenstrom (D") so bemißt, daß dabei ein möglichst weitgehender Übergang des in (D") enthaltenen Polyamins in die die Extraktionsstufe (2) verlassende wäßrige Phase stattfindet, besagte wäßrige Phase, aus Mengenstrom (Y) und/oder Hilfsamin und/oder weiterer wäßriger Phase aus Mengenstrom (C) der Extraktionsstufe (3) zuführt, die in (2) anfallende organische Phase (Z), bestehend im wesentlichen aus hydrophobem Lösungsmittel und Hilfsamin, ebenfalls der Extraktionsstufe (3) zuführt und als Bestandteil der organischen Phase (B) als Lösungsmittel für das Ausgangspolyamin (A) verwendet.

4. Verfahren gemäß einem oder mehreren der Ansprüche 1 bis 3, dadurch gekennzeichnet, daß der wiederverwendete Mengenstrom (C) einen höheren Protonierungsgrad aufweist als die die Extraktionsstufe (4) verlassende wäßrige Phase (K), gegebenenfalls bis zu 100 %.

5. Verfahren gemäß einem oder mehreren der Ansprüche 1 bis 4, dadurch gekennzeichnet, daß man

f) die die Extraktionsstufe (5) verlassende wäßrige Phase (K) vor ihrer Wiederverwendung zumindest teilweise und/oder die die Extraktionsstufe (6) verlassende wäßrige Phase (Q) vor ihrer Wiederverwendung zumindest teilweise destillativ (9) von einem Teil (X) des in ihr enthaltenen Wassers befreit, dieses zum Waschen (7.0) des der destillativen Aufarbeitung (7.1), (7.2) zugeführten Teiles der die Extraktionsstufe (3) verlassenden organischen Phase (D) und/oder zum Waschen (8.0) des der destillativen Aufarbeitung (8.1), (8.2) zugeführten Teiles der die Extraktionsstufe (5) verlassenden organischen Phase (L) zwecks Entfernens von Säurespuren verwendet, das hierbei anfallende Wasser (Y) an geeigneter Stelle in die wäßrige Phase zurückführt, die resultierende konzentrierte wäßrige Phase mit dem gegebenenfalls verbliebenen Resten von (K) und (Q) vereinigt und als Mengenstrom (C) der Wiederverwendung zuführt.

6. Verfahren gemäß einem oder mehreren der Ansprüche 1 bis 5, dadurch gekennzeichnet, daß man als Hilfsamin Anilin verwendet.

7. Verfahren gemäß einem oder mehreren der Ansprüche 1 bis 6, dadurch gekennzeichnet, daß man die die Extraktionsstufe (3) verlassende organische Phase (D) eine Waschstufe (7.0) und/oder die die Extraktionsstufe (5) verlassende organische Phase (L) eine Waschstufe (8.0) durchlaufen läßt.

8. Verfahren gemäß einem oder mehreren der Ansprüche 1 bis 7, dadurch gekennzeichnet, daß man die die Extraktionsstufe (3) verlassende wäßrige Phase (H) zumindest teilweise über eine zwischengeschaltete Extraktionsstufe (4) und/oder die in der Extraktionsstufe (5) anfallende wäßrige Phase (K) zumindest teilweise über eine zwischengeschaltete Destillationsstufe (9) und/oder die wäßrige Phase (Q) zumindest teilweise über eine zwischengeschaltete Destillationsstufe (9) laufen läßt.

9. Verfahren gemäß einem oder mehreren der Ansprüche 1 bis 8, dadurch gekennzeichnet, daß man ein Polyamingemisch der Diphenylmethanreihe als Polyamingemisch verwendet, wie es bei der säurekatalysierten Anilin/Formaldehydkondensation anfällt.

10. Verfahren zur Herstellung von entsprechenden aromatischen Polyisocyanatgemische, dadurch gekennzeichnet, daß gemäß den Ansprüchen 1 bis 9 behandelte aromatische Polyamingemische verwendet werden.

11. Verfahren zur Herstellung kernhydrierter Polyamine oder Vernetzer und Epoxidhärter, dadurch gekennzeichnet, daß gemäß den Ansprüchen 1 bis 9 behandelte aromatische Polyamingemische verwendet werden.

12. Verfahren zur Herstellung von Polyurethankunststoffen, dadurch gekennzeichnet, daß gemäß den Ansprüchen 1 bis 7 behandelte aromatische Polyamingemische verwendet werden.

**Claims**

1. Process for the fractionation and purification of aromatic polyamine mixtures of the diphenylmethane series, characterised in that

a) the polyamine starting mixture (A) is distributed in a two-phase system consisting of (i) a hydrophobic solvent phase (B), which consists substantially of hydrophobic solvent, and (ii) an aqueous phase (C) consisting substantially of water, a strong acid and auxiliary amine, which amine is present at least partly in the salt form, is virtually insoluble in water and has a boiling point which under normal pressure is at least 20°C below the boiling point of the lowest-boiling component of the starting mixture and at least 20°C above the boiling point of the solvent, by means of an extraction step (3) operating according to the counter-current principle, with thorough mixing of the phases, by introducing the starting polyamine mixture into the extraction step (3), and the organic phase (D) leaving the extraction step, at least partly, in a multi-step distillation (7.1), (7.2), is separated into a first fraction (E) which consists substantially of hydrophobic solvent and is reused in the extraction step (3), a second fraction (F) consisting substantially of auxiliary amine and a distillation residue (G) consisting substantially of a first polyamine fraction, and the aqueous phase (H) leaving the extraction step (3)

b) is passed to an extraction step (5), in which an extraction of the aqueous phase with a solvent phase (J) consisting of hydrophobic solvent and auxiliary amine is carried out according to the principle of counter-current extraction, the aqueous phase (K) depleted of polyamine being obtained as a result, and

c) the organic phase (L) accumulating in the extraction step (5), at least partly, in a multi-step distillation (8.1), (8.2), is separated into a first fraction (M) consisting substantially of hydrophobic solvent and a second fraction (N) consisting substantially of auxiliary amine and a distillation residue (O) consisting substantially of a second polyamine fraction, and

d) the aqueous phase (K) accumulating in the extraction step (5), at least partly,

e) is passed to an extraction step (6) and is extracted in counter-current with an organic phase (P) consisting substantially of hydrophobic solvent and at most only so much auxiliary amine that in (6) an aqueous phase (Q) which is depleted of arylamine in comparison with the added aqueous phase (K) is obtained as a result, which phase (Q)

f) is reused as mass flow (C), by passing mass flow (C)

g) to the extraction step (3) and

g) the organic phase (R) accumulating in the extraction step (6), as extracting agent (J), is passed to the extraction step (5).

2. Process according to claim 1, characterised in that

b) the aqueous phase (H) accumulating in the extraction step (3) is at least partly extracted in a tandem-arranged extraction step (4) operating according to the counter-current principle, using as extracting agent an organic phase (T), consisting of hydrophobic solvent and additionally of auxiliary amine and polyamine, the latter introduced as a part quantity of the mass flow (L), the resulting organic phase (U) in the process step (4) is passed to the mass flow (B) and with it to the extraction step (3) and the resulting aqueous phase (V) is passed to the extraction step (5).

**3.** Process according to claim 1 or 2, characterised in that

j) a part quantity (D") of the organic phase (D) leaving the extraction step (3) is separated off and extracted in counter-current with the entire mass flow (C) in a preferably multi-step extraction step (2) placed in front, optionally with addition of auxiliary amine, and the partial mass flow (D") is so calculated that here as extensive a transition as possible of the polyamine present in (D") into the aqueous phase leaving the extraction step (2) takes place, the said aqueous phase, consisting of mass flow (Y) and/or auxiliary amine and/or other aqueous phase consisting of mass flow (C) is passed to the extraction step (3), the organic phase (Z) accumulating in (2), consisting substantially of hydrophobic solvent and auxiliary amine, is likewise passed to the extraction step (3) and as a component of the organic phase (B) is used as solvent for the starting polyamine (A).

**4.** Process according to one or more of claims 1 to 3, characterised in that the reused mass flow (C) has a degree of protonation higher than that of the aqueous phase (K) leaving the extraction step (4), if necessary up to 100%.

**5.** Process according to one or more of claims 1 to 4, characterised in that

f) before being reused the aqueous phase (K) leaving the extraction step (5) and/or the aqueous phase (Q) leaving the extraction step (6), is freed at least partially from a part (X) of the water contained therein by distillation (9), this water is used for washing (7.0) the portion of the organic phase (D) leaving the extraction step (3) and fed to the working-up by distillation (7.1), (7.2) and/or for washing (8.0) the portion of the organic phase (L) leaving the extraction step (5) and fed to the working-up by distillation (8.1), (8.2), in order to remove traces of acid, the water (Y) accumulating here is returned to the aqueous phase at a suitable point, the resulting concentrated aqueous phase is combined with any possibly remaining traces of (K) and (Q) and returned for reuse as mass flow (C).

**6.** Process according to one or more of claims 1 to 5, characterised in that aniline is used as auxiliary amine.

**7.** Process according to one or more of claims 1 to 6, characterised in that the organic phase (D) leaving the extraction step (3) is passed through a washing step (7.0) and/or the organic phase (L) leaving the extraction step (5) is passed through a washing step (8.0).

**8.** Process according to one or more of claims 1 to 7, characterised in that the aqueous phase (H) leaving the extraction step (3) is passed at least partly through an intermediate extraction step (4) and/or the aqueous phase (K) accumulating in the extraction step (5) is passed at least partly through an intermediate distillation step (9) and/or the aqueous phase (Q) is passed at least partly through an intermediate distillation step (9).

**9.** Process according to one or more of claims 1 to 8, characterised in that the polyamine mixture used is a polyamine mixture of the diphenylmethane series such as is obtained in the acid-catalysed aniline/formaldehyde condensation.

**10.** Process for the preparation of the corresponding aromatic polyisocyanate mixtures, characterised in that aromatic polyamine mixtures treated according to claims 1 to 9 are used.

**11.** Process for the preparation of ring-hydrogenated polyamines or cross-linking agents and epoxy curing agents, characterised in that aromatic polyamine mixtures treated according to claims 1 to 9 are used.

**12.** Process for the production of polyurethane plastics, characterised in that aromatic polyamine mixtures treated according to claims 1 to 7 are used.

**Revendications**

**1.** Procédé pour fractionner et purifier des mélanges de polyamines aromatiques de la série du diphénylméthane, caractérisé en ce que

a) on partage le mélange des polyamines de départ (A) dans un système à deux phases consistant en (i) une phase de solvant hydrophobe (B) qui consiste elle-même essentiellement en un solvant hydrophobe, et (ii) une phase aqueuse (C) consistant essentiellement en eau, un acide fort et une amine auxiliaire présente en partie au moins à l'état de sel, cette amine étant pratiquement insoluble dans l'eau et ayant, à pression normale, un point d'ébullition situé à au moins 20°C au-dessous du point d'ébullition du composant bouillant le plus bas du mélange initial et à au moins 20°C au-dessus du point d'ébullition du solvant, grâce à un stade d'extraction (3) opérant selon le principe du contre-courant, avec mélange des phases, en introduisant le mélange des polyamines de départ au stade d'extraction (3) et en séparant la phase organique (D) quittant le stade d'extraction, en partie au moins, dans une distillation à plusieurs étages (7.1), (7.2), en une première fraction (E), réutilisée au stade d'extraction (3) et qui consiste essentiellement en le solvant hydrophobe, une deuxième fraction (F) consistant essentiellement en l'amine auxiliaire et un résidu de distillation (G) consistant essentiellement en une première fraction de polyamines,

b) on envoie la phase aqueuse (H) quittant le stade d'extraction (3), dans un stade d'extraction (5) dans lequel, selon le principe de l'extraction à contre-courant, on extrait la phase aqueuse par une phase solvant (J) consistant en le solvant hydrophobe et l'amine auxiliaire, ce qui donne une phase aqueuse (K) appauvrie en polyamines, et

c) on sépare la phase organique (L) obtenue au stade d'extraction (5), en partie au moins, dans une distillation à plusieurs étages (8.1), (8.2), en une première fraction (M) consistant essentiellement en le solvant hydrophobe et une deuxième fraction (N) consistant essentiellement en l'amine auxiliaire, et un résidu de distillation (O) consistant essentiellement en une deuxième fraction de polyamines, et

d) on envoie la phase aqueuse (K) obtenue au stade d'extraction (5), en partie au moins,

(e) dans un stade d'extraction (6) et on l'extrait à contre-courant par une phase organique (P) consistant essentiellement en le solvant hydrophobe et l'amine auxiliaire en quantité juste suffisante pour que, dans (6), on obtienne une phase aqueuse (Q) appauvrie en arylamines par rapport à la phase aqueuse mise en oeuvre (K),

(f) on réutilise la phase aqueuse (Q) en tant que courant (C)

(g) qu'on envoie au stade d'extraction (3) et

(h) on envoie la phase organique (R) sortant du stade d'extraction (6), en tant que phase agent d'extraction (J) au stade d'extraction (5).

2. Procédé selon la revendication 1, caractérisé en ce que

b) on extrait la phase aqueuse (H) obtenue au stade d'extraction (3) en partie au moins dans un stade d'extraction successif (4), opérant selon le principe du contre-courant, en utilisant en tant qu'agent d'extraction une phase organique (T) qui consiste en le solvant hydrophobe, l'amine auxiliaire et des polyamines, ces dernières introduites en tant que partie du courant (L), on envoie la phase organique (Q) obtenue au stade opératoire (4) vers le courant (B) et par conséquent au stade d'extraction (3) et on envoie la phase aqueuse (V) obtenue dans ce dernier stade au stade d'extraction (5).

3. Procédé selon la revendication 1 ou 2, caractérisé en ce que

j) on sépare une partie (D") de la phase organique (D) quittant le stade d'extraction (3) et on l'extrait dans un stade d'extraction préalable (2), de préférence à plusieurs étages, par la totalité du courant (C), à contre-courant, le cas échéant avec adjonction de l'amine auxiliaire, et on dose le courant (D") en sorte qu'il se produise un transfert aussi complet que possible des polyamines contenues dans (D") vers la phase aqueuse quittant le stade d'extraction (2), on envoie ladite phase aqueuse, consistant en le courant (Y) et/ou l'amine auxiliaire et/ou un complément de phase aqueuse du courant (C), au stade d'extraction (3), on envoie la phase organique (Z) obtenue en (2) et qui consiste essentiellement en le solvant hydrophobe et l'amine auxiliaire, également au stade d'extraction (3), et on l'utilise en tant que constituant de la phase organique (B), en tant que solvant pour les polyamines de départ (A).

4. Procédé selon une ou plusieurs des revendications 1 à 3, caractérisé en ce que le courant réutilisé (C) est à un taux de protonation supérieur à celui de la phase aqueuse (K) quittant le stade d'extraction (4), et qui peut le cas échéant atteindre 100 %.

5. Procédé selon une ou plusieurs des revendications 1 à 4, caractérisé en ce que

f) la phase aqueuse (K) quittant le stade d'extraction (5), avant sa réutilisation, et/ou la phase aqueuse (Q) quittant le stade d'extraction (6), avant sa réutilisation, sont débarrassées, en partie au moins, par distillation (9)

d'une partie (X) de l'eau qu'elles contiennent, celle-ci est utilisée pour le lavage de la partie de la phase organique (D) quittant le stade d'extraction (3) et envoyée à la distillation (7.1), 7.2) et/ou de la partie de la phase organique (L) quittant le stade d'extraction (5) envoyée à la distillation (8.1), (8.2) en vue d'éliminer les traces d'acide, l'eau (Y) ainsi obtenue est recyclée dans la phase aqueuse à un endroit quelconque, la phase aqueuse concentrée résultante est combinée avec les résidus éventuels de (K) et (Q) et envoyée en tant que courant (C) à la réutilisation.

**6.** Procédé selon une ou plusieurs des revendications 1 à 5, caractérisé en ce que l'amine auxiliaire utilisée est l'aniline.

**7.** Procédé selon une ou plusieurs des revendications 1 à 6, caractérisé en ce que la phase organique (D) quittant le stade d'extraction (3) passe par un stade lavage (7.0) et/ou la phase organique (L) quittant le stade d'extraction (5) passe par un stade lavage (8.0).

**8.** Procédé selon une ou plusieurs des revendications 1 à 7, caractérisé en ce que la phase aqueuse (H) quittant le stade d'extraction (3) passe en partie au moins par un stade d'extraction intermédiaire (4) et/ou la phase aqueuse (K) quittant le stade d'extraction (5) passe en partie au moins par un étage de distillation intermédiaire (9) et/ou la phase aqueuse (Q) passe en partie au moins par un étage intermédiaire de distillation (9).

**9.** Procédé selon une ou plusieurs des revendications 1 à 8, caractérisé en ce que le mélange de polyamines de départ consiste en un mélange de polyamines de la série du diphénylméthane tel qu'obtenu par condensation aniline/formaldéhyde avec catalyse acide.

**10.** Procédé pour la préparation de mélanges de polyisocyanates aromatiques correspondants, caractérisé en ce que l'on utilise des mélanges de polyamines aromatiques traités selon les revendications 1 à 9.

**11.** Procédé pour la préparation de polyamines hydrogénées dans les noyaux ou d'agents réticulants et de durcisseurs de résines époxydiques, caractérisé en ce que l'on utilise des mélanges de polyamines aromatiques traités selon les revendications 1 à 9.

**12.** Procédé pour la préparation de résines synthétiques de polyuréthannes, caractérisé en ce que l'on utilise des mélanges de polyamines aromatiques traités selon les revendications 1 à 7.

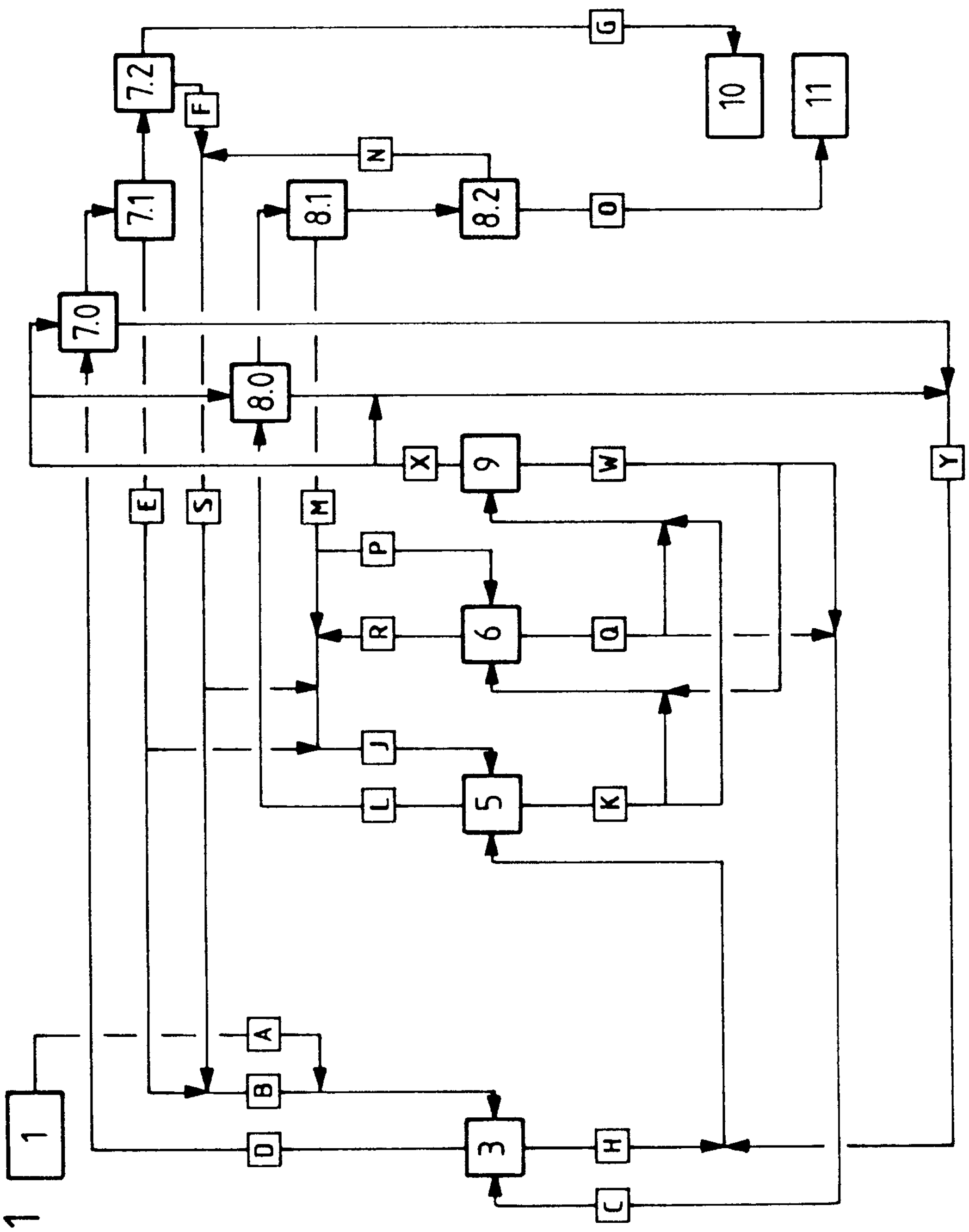


Fig.1

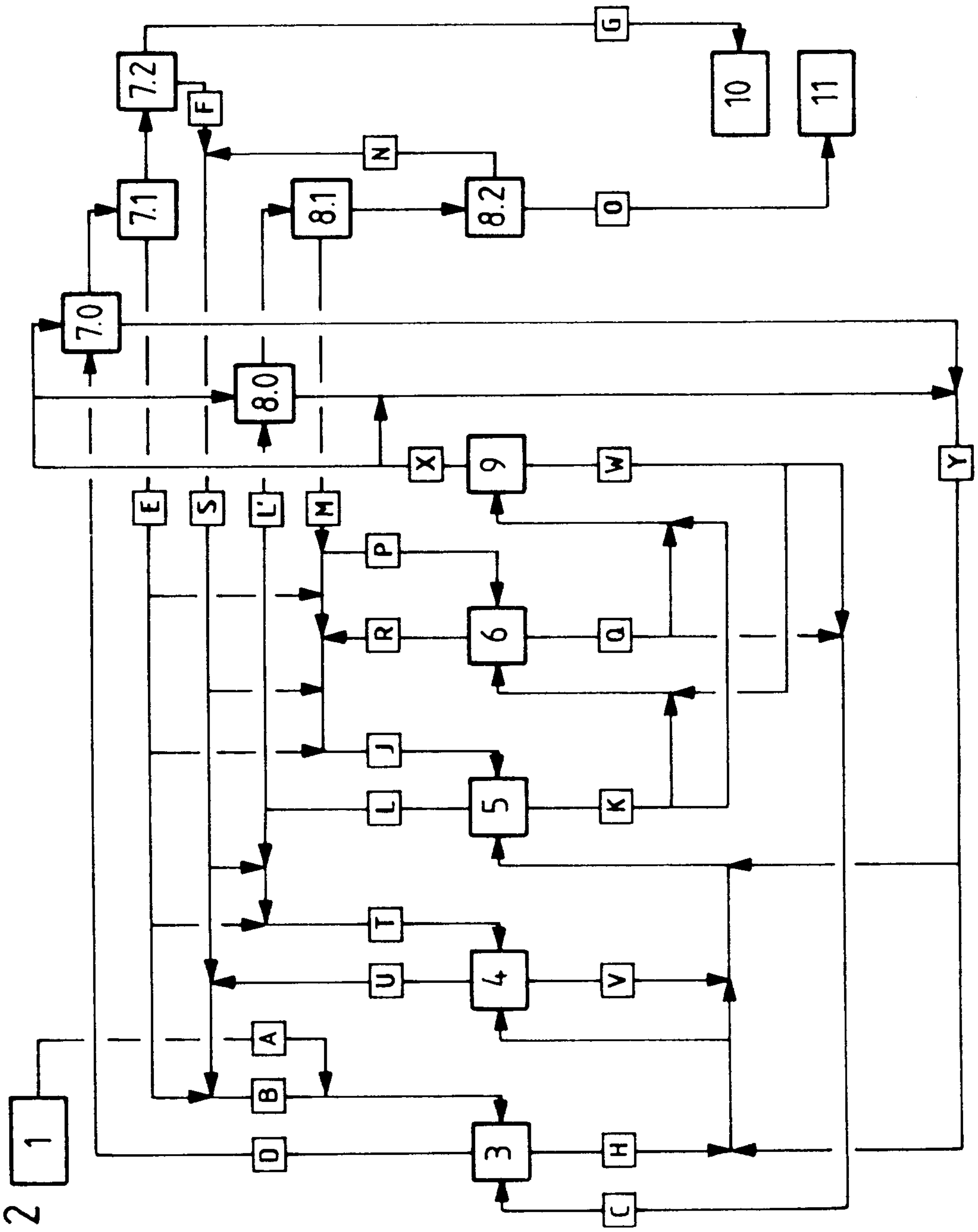


Fig. 2

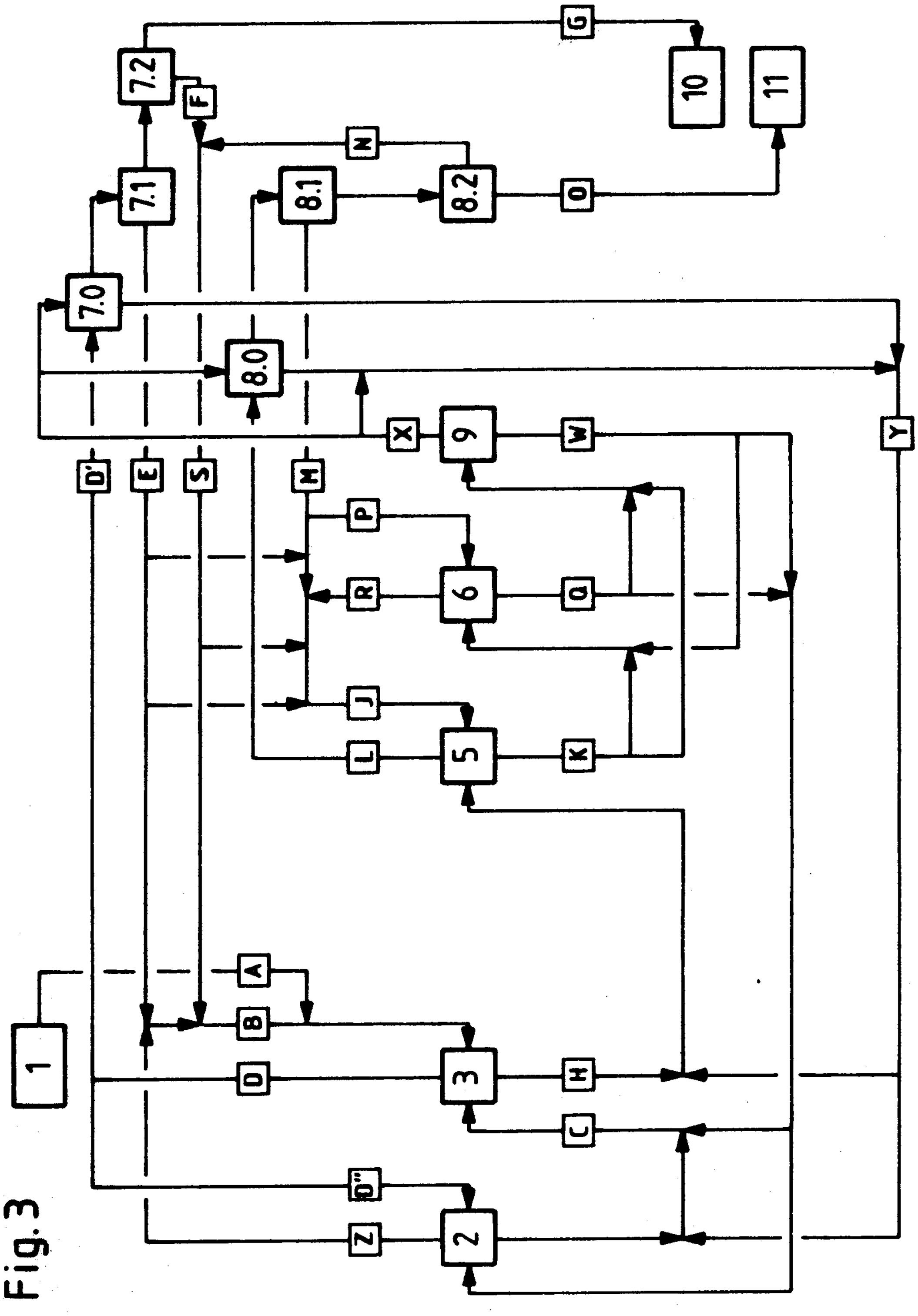


Fig.3

Fig. 4

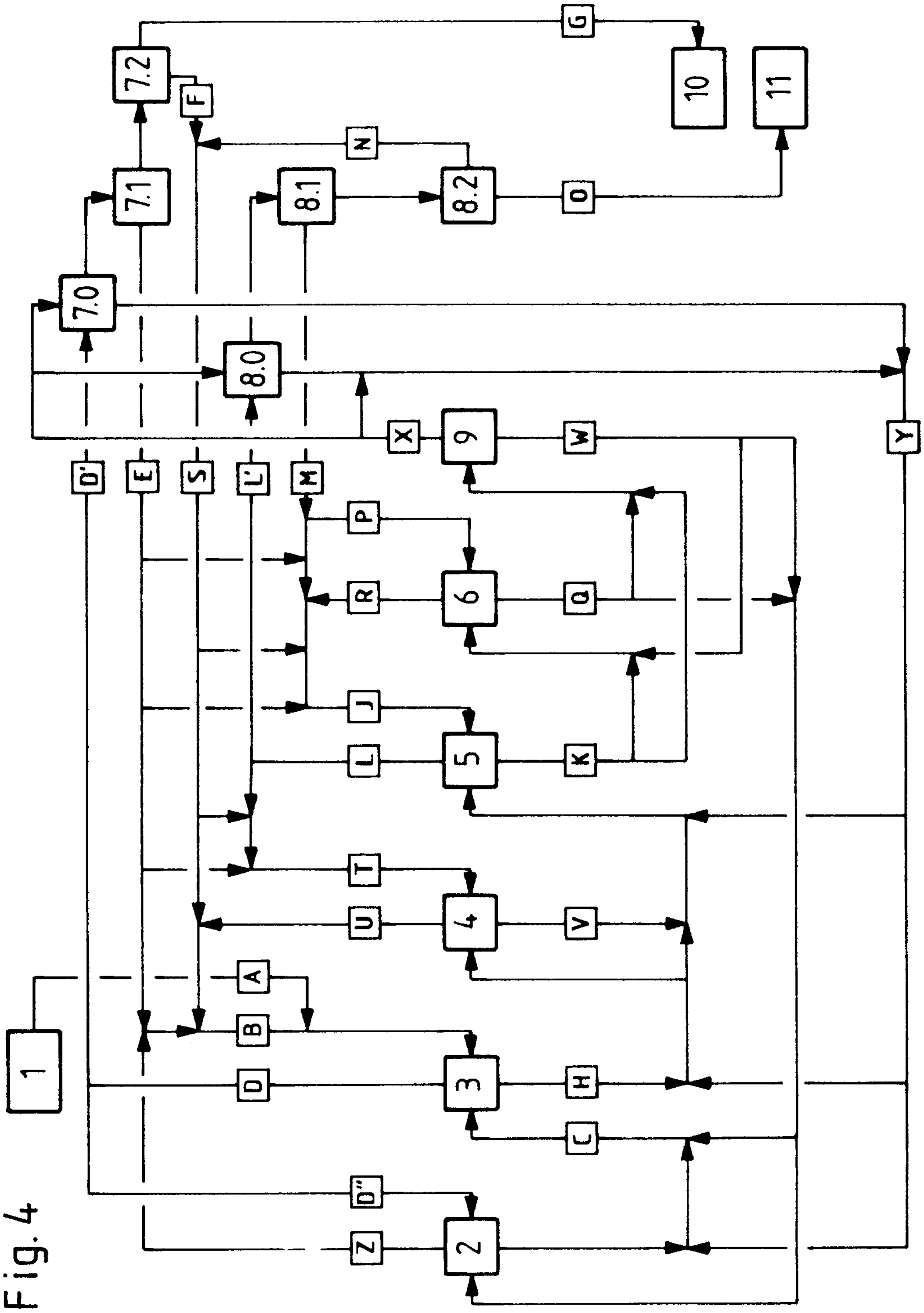
1
2
3
4
5
6
7.0
7.1
7.2
8.0
8.1
8.2
9
10
11
A
B
C
D
D'
D''
E
F
G
H
J
K
L
L'
M
N
O
P
Q
R
S
T
U
V
W
X
Y
Z